# EUROPEAN PATENT SPECIFICATION

(11) **EP 1 618 828 B1**
(45) Date of publication and mention of the grant of the patent: **02.05.2012**
(21) Application number: 04728669.5
(22) Date of filing: 21.04.2004
(51) Int. Cl.: A61B 1/00

(54) **DEVICE, METHOD AND PROGRAM FOR IMAGE PROCESSING**
VORRICHTUNG, VERFAHREN UND PROGRAMM FÜR DIE BILDBEARBEITUNG
DISPOSITIF, PROCEDE ET PROGRAMME POUR TRAITEMENT D'IMAGES

(30) Priority: 25.04.2003 JP 2003122804; 15.04.2004 JP 2004120367
(43) Date of publication of application: 25.01.2006
(60) Divisional application: 07017743.1
(73) Proprietor: Olympus Corporation, Tokyo 151-0072 (JP)
(72) Inventor: HONDA, Takemitsu c/o Olympus Med. Syst. Corp., Tokyo 151-0072 (JP); MINAI, Tetsuo, 1920023 (JP)
(74) Representative: von Hellfeld, Axel
(86) International application number: PCT/JP2004/005738
(87) International publication number: WO 2004/096025

(56) References cited:
- WO-A1-02/21530
- WO-A2-02/073507
- JP-A- 11 024 649
- JP-A- 11 225 996
- JP-A- 2002 290 783
- US-A- 5 781 188
- US-A1- 2002 171 669

## Description

### TECHNICAL FIELD

The present invention relates to an image display apparatus, an image display method, and an image display program.

### BACKGROUND ART

Recently, swallowable capsule endoscopes have been produced as a type of endoscopes. The capsule endoscopes are provided with an imaging capability and a radio capability. A capsule endoscope is configured to sequentially take images of organs such as the stomach and the small intestine within an observation period from the time it has been swallowed through the mouth of a patient for observation (examination) to its natural excretion from the human body (see Japanese Patent Application Laid-open No. H11-225996).

During the observation period, image data taken in a body by the capsule endoscope is sequentially transmitted outside through radio communication and is stored in a memory. Since a patient carries around a receiver having a radio communication capability and a memory capability, the patient can freely perform normal actions during the observation period from swallowing of the capsule endoscope to its excretion. After observation, a doctor or a nurse can display the images of organs on a display based on the image data stored in the memory and use it to make a diagnosis.

As the above type of capsule endoscope, "M2A (registered trademark)" by Predetermined Imaging Ltd. of Israel, and "NORIKA (registered trademark)" by RF SYSTEM lab. of Japan are presently available, and they have already come to practical applications.

However, unlike an ordinary endoscope, the capsule endoscope described above takes images of each organ within a period from the time a subject swallows to its natural excretion, meaning an extended period of observation (examination), for example, not less than ten hours. Therefore, the number of images to be taken in time sequence is correspondingly huge.

At the stage of diagnosis or the like, no particular consideration is predetermined to improving the ability to retrieve a desired image from the vast amount of images taken over a long period of time, or providing a display screen allowing easy recognition of what time in the overall imaging period the displayed image was taken, of which organ is being shown, and the like.

Document US 2002/171669 A1 concerns an image display apparatus for receiving and displaying image data from a capsule endoscope. This document discloses a timeline that can be associated with annotations.

WO 02/21530 A1 concerns a reproducing apparatus for displaying a colored slide bar for browsing a video. According to one embodiment, the average color of an image of the video is displayed in a portion of the slide bar corresponding to the image.

Document JP 11-24649 A concerns a method for representing a function in a display portion by means of a color bar

The object of the present invention is to provide an image display apparatus, image display method, and image display program which can improve the ability to retrieve taken internal images of a body and ensure easy recognition of the organ depicted in each image.

### DISCLOSURE OF INVENTION

Some or all of the the above problems are overcome by an image display apparatus according to claim 1, an image display method according to claim 5, and an image display program according to claim 9.

To solve the above problems and to realize the object, an image display apparatus according to the present invention includes an input unit that inputs image data taken in time sequence by an in-vivo imaging device; a scale display control unit that controls to display a scale indicating an overall imaging period of the image data taken in time sequence and input by the input unit and to display a movable slider on the scale; an image display control unit that controls to display on a display unit an image at an imaging time corresponding to a position of the slider in response to a movement of the slider on the scale; a color information detecting unit that detects color information of a screen of the image data input by the input unit; and a color display control unit that controls to display a color corresponding to the color information detected by the color information detecting unit at position on the scale corresponding to time.

In the image display apparatus according to the above invention, the color information detecting unit may include an average color detecting unit that detects color information on an average color from color information of the screen of the image data input by the input unit.

The image display apparatus according to the above invention may further include an organ discriminating unit that discriminates an organ based on the color information detected by the color information detecting unit; and an organ name display control unit that controls to display a name of the organ discriminated in accordance with the scale.

In the image display apparatus according to the above invention, the organ discriminating unit may discriminate the organ based on information on increase and decrease of color elements constituting the color information.

In the image display apparatus according to the above invention, the organ discriminating unit may discriminate the organ further based on in-vivo information acquired in accordance with the image data.

An image display apparatus according to the present invention includes a characteristic extracting unit that extracts a numerical parameter that characterizes an image taken in a time sequence by an in-vivo imaging device; and a display control unit that visualizes the numerical parameter extracted by the characteristic extracting unit, and displays the numerical parameter visualized time-sequentially and continuously.

In the image display apparatus according to the above invention, the numerical parameter may be a color element indicating an average color of each image.

The image display apparatus according to the above invention may further include a converting unit that converts the numerical parameter extracted into a new numerical parameter, and the display control unit may visualize the new numerical parameter converted by the converting unit and displays the new numerical parameter time-sequentially and continuously.

According to the above aspect of the present invention, the converting unit may convert the numerical parameter extracted into a new numerical parameter indicating a value of luminance of each image based on a numerical parameter of color information of each image.

In the image display apparatus according to the above invention, the converting unit may convert the numerical parameter extracted into a numerical parameter indicating a value of average luminance of each image based on a numerical parameter of an average color of each image.

In the image display apparatus according to the above invention, the characteristic extracting unit may extract an inter-frame error indicating a difference between individual image frames as the numerical parameter.

The image display apparatus according to the above invention may further include an internal organ discriminating unit that discriminates an organ based on the numerical parameter extracted or the new numerical parameter converted, and the display control unit may control to display the organ discriminated corresponding to the time sequence.

The image display apparatus according to the above invention further includes an input unit that inputs image data taken in time sequence by an internal imaging device; a scale display control unit that controls to display a scale indicating an overall imaging period of the image data taken in time sequence and input by the input unit and to display a movable slider on the scale; and an image display control unit that controls to display on a display unit an image at an imaging time corresponding to a position of the slider in response to a movement of the slider on the scale. The display control unit controls to visualize the numerical parameter extracted by the characteristic extracting unit or the new numerical parameter converted by the converting unit, and display the visualized numerical parameter at a position on the scale corresponding to the time sequence.

An image display apparatus according to the present invention includes a color information acquiring unit that acquires color information of each image in a series of image data taken in time sequence by an in-vivo imaging device; a converting unit that converts the color information acquired by the color information acquiring unit to positional information on a predetermined characteristic space; a bleeding part determining unit that determines whether a bleeding part to be detected is present in an image based on color distribution positional information on bleeding in the predetermined characteristic space and the positional information converted by the converting unit; and a flag marking unit that marks a flag indicating presence of a bleeding part to be searched to an image that is determined to include the bleeding part by the bleeding part determining unit.

In the image display apparatus according to the above invention, the bleeding part determining unit may determine a type of bleeding by using an identification function that separates color distributions of bright red blood, coagulated blood, and normal bleeding on the characteristic space.

In the image display apparatus according to the present invention, when a bleeding part is nearly circular, the bleeding part determining unit may determine that the bleeding part is a coagulated blood bleeding part.

In the image display apparatus according to the above invention, the bleeding part determining unit may detect an edge of each pixel in the image, generate normal lines to each edge detected, and when the number of normal lines intersecting each pixel is equal to or greater than a predetermined value, determine that there is a nearly circular bleeding part.

An image display method and an image display program according to the present invention each include an input step for inputting image data taken in time sequence by an in-vivo imaging device; a scale display control step for controlling to display a scale indicating an overall imaging period of the image data taken in time sequence and input in the input step and to display a movable slider on the scale; an image display control step for controlling to display on a display unit an image at an imaging time corresponding to a position of the slider in response to a movement of the slider on the scale; a color information detecting step for detecting color information of a screen of the image data input in the input step; and a color display control step for controlling to display a color corresponding to the color information detected in the color information detecting step at position on the scale corresponding to time.

In each of the image display method and the image display program according to the above invention, the color information detecting step may include an average color detecting step for detecting the color information on an average color from color information of the screen of the image data input.

The image display method and the image display program according to the above invention each may further include an organ discriminating step for discriminating an organ based on the color information detected in the color information detecting step; and an organ-name display control step for controlling to display a name of the organ discriminated in the organ discriminating step in accordance with the scale.

In each of the image display method and the image display program according to the above invention, the organ discriminating step may include discriminating the organ based on information on increase and decrease of color elements constituting the color information.

In each of the image display method and the image display program according to the above invention, the organ discriminating step may include discriminating the organ further based on in-vivo information acquired in accordance with the image data.

An image display method and an image display program according to the present invention each include a characteristic extracting step for extracting a numerical parameter that characterizes an image taken in a time sequence by an in-vivo imaging device; a display control step for visualizing the numerical parameter extracted in the characteristic extracting step to display the visualized numerical parameter time-sequentially and continuously.

In each of the image display method and the image display program according to the above invention, the numerical parameter may be a color element indicating an average color of each image.

Each of the image display method and the image display program according to the above invention may further include a converting step for converting the numerical parameter extracted in the characteristic extracting step into a new numerical parameter, and the display control step may include visualizing the new numerical parameter converted in the converting step to display the new numerical parameter time-sequentially and continuously.

In each of the image display method and the image display program according to the above invention, the converting step may include converting the-numerical parameter extracted into a new numerical parameter indicating a value of luminance of each image based on a numerical parameter of color information of each image.

In each of the image display method and the image display program according to the above invention, the converting step may include converting the numerical parameter extracted into a numerical parameter indicating a value of average luminance of each image based on a numerical parameter of an average color of each image.

In each of the image display method and the image display program according to the above invention, the characteristic extracting step may include extracting an inter-frame error indicating a difference between individual image frames as the numerical parameter.

Each of the image display method and the image display program according to the above invention may further include an organ discriminating step for discriminating an organ based on the numerical parameter extracted or the new numerical parameter converted, and the display control step may include controlling to display the organ discriminated corresponding to the time sequence.

Each of the image display method and the image display program according to the above invention further includes an inputting step for inputting image data taken in time sequence by an internal imaging device; a scale display control step for displaying a scale indicating an overall imaging period of the image data taken in time sequence and input in the inputting step to control to display a movable slider on the scale; and an image display control step for controlling to display on a display unit an image at an imaging time corresponding to a position of the slider in response to a movement of the slider on the scale. The display control step includes visualizing the numerical parameter extracted in the characteristic extracting step or the numerical parameter converted in the converting step to control to display the visualized numerical parameter at a position on the scale corresponding to the time sequence.

An image display method and an image display program according to the present invention each include a color information acquiring step for acquiring color information of each image in a series of image data taken in time sequence by an in-vivo imaging device; a converting step for converting the color information acquired in the color information acquiring step to positional information on a predetermined characteristic space; a bleeding part determining step for determining whether a bleeding part to be detected is present in an image based on color distribution positional information on bleeding in the predetermined characteristic space and the positional information converted in the converting step; and a flag marking step for marking a flag indicating presence of a bleeding part to be searched to an image that is determined to include the bleeding part in the bleeding part determining step.

Each of the image display method and the image display program according to the above invention, the bleeding part determining step may include determining a type of bleeding by using an identification function that separates color distributions of bright red blood, coagulated blood, and normal bleeding on the characteristic space.

In each of the image display method and the image display program according to the above invention, the bleeding part determining step may include determining that the bleeding part is a coagulated blood bleeding part when a bleeding part is nearly circular.

In each of the image display method and the image display program according to the above invention, the bleeding part determining step may include detecting an edge of each pixel in the image; generating normal lines to each edge detected, and determining that there is a nearly circular bleeding part when number of the normal lines intersecting each pixel is equal to or greater than a predetermined value.

### BRIEF DESCRIPTION OF DRAWINGS

Fig. 1 is a schematic diagram of the internal structure of a capsule endoscope according to an illustrative example of the present invention;
Fig. 2 is a schematic diagram of a capsule endoscope system according to the illustrative example ;
Fig. 3 is a block diagram showing a structural example of the capsule endoscope system according to the illustrative example ;
Fig. 4 shows an example of screen transition associated with the observation procedures according to the illustrative example ;
Fig. 5 shows an example of screen transition associated with the observation procedures according to the illustrative example ;
Fig. 6 shows an example of screen transition associated with the observation procedures according to the illustrative example
Fig. 7 shows an example of screen transition associated with the diagnosis procedures according to the illustrative example ;
Fig. 8 shows an example of screen transition associated with the diagnosis procedures according to the illustrative example ;
Fig. 9 is a flowchart of procedures of an automatic bleeding part searching process;
Fig. 10 is a diagram of the characteristic space and the relationship among a bright red blood area, coagulated blood area, and a normal bleeding area and ID functions on the characteristic space;
Fig. 11 is a detailed flowchart of procedures of image processing for a circle shown in Fig. 9;
Fig. 12 is a schematic for illustrating an image processing for detecting a circle by edge detection;
Fig. 13 is a flowchart of an operation for displaying average color bar;
Fig. 14 shows an example of a display screen associated with a diagnosis process according to a modification of the illustrative example ;
Fig. 15 shows the principle of automatic discrimination of organ names according to the modification of the illustrative example ;
Fig. 16 is a flowchart of the procedures of discriminating the organ names according to the modification of the illustrative example ;
Fig. 17 shows an example of application of the modification shown in Fig. 15; Fig. 18 shows a display state of average color elements of individual images in a time-sequential order;
Fig. 19 shows a display state of average luminance acquired from the average color elements of individual images in a time-sequential order;
Fig. 20 shows a display state of inter-frame errors of individual images in a time-sequential order;
Fig. 21 shows an example of screen transition associated with the diagnosis procedures according to the illustrative example; and
Fig. 22 is a flowchart of an operation for displaying the imaging time of a designated image according to the illustrative example.

### BEST MODE(S) FOR CARRYING OUT THE INVENTION

Illustrative examples of the present invention are described below with reference to the accompanying drawings.

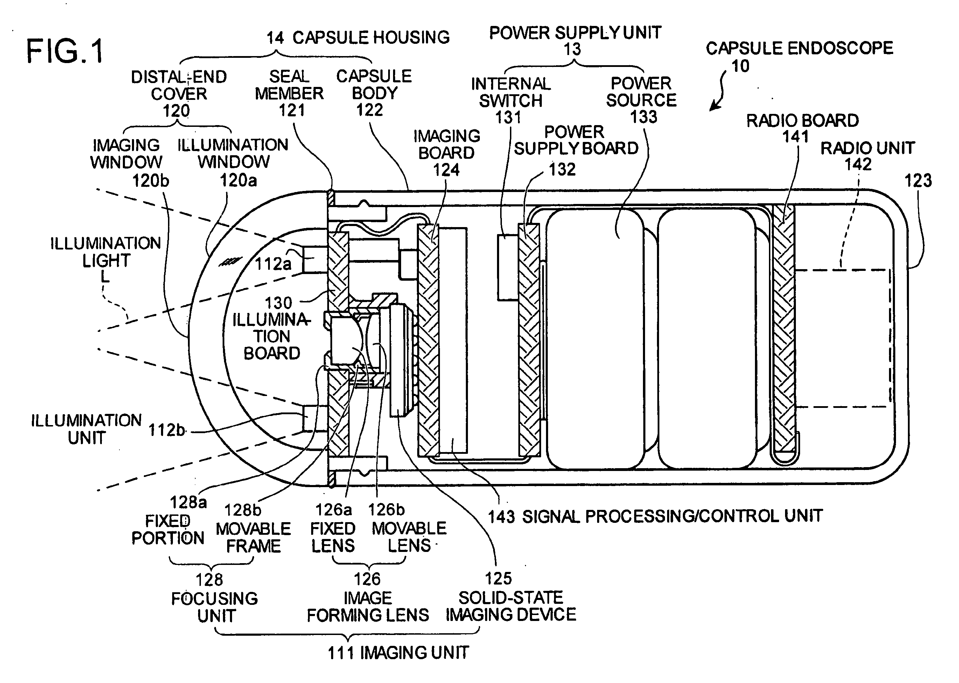

The overall structure of a capsule endoscope according to one illustrative example of the present invention is described with reference to Fig. 1. Fig. 1 is a schematic diagram of the internal structure of a capsule endoscope according to an illustrative example of the present invention. As shown in Fig. 1, an capsule endoscope 10 includes an imaging unit 111 which can take the internal image of a celom, illumination units 112a and 112b which illuminate the interior of the celom, a power supply unit 13 which supplies them with power, and a capsule housing 14 which has at least the imaging unit 111, the illumination units 112 and the power supply unit 13 disposed inside.

The capsule housing 14 according to the present illustrative example includes a distal-end cover 120 which covers the imaging unit 111 and the illumination units 112a, 112b, and a capsule body 122 which is provided in a water-proof state with respect to the distal-end cover 120 via a seal member 121 and has the imaging unit 111, etc. disposed therein. A rear-end cover 123 may be provided as separate from the capsule body 122 as needed. Although the rear-end cover 123 is provided integrally with the capsule body and has a flat shape in the present illustrative example, the shape is not limited and may be, for example, a dome shape.

The distal-end cover 120 may clearly separate an illumination window 120a, which transmits illumination light L from the illumination unit 112a, 112b, and an imaging window 120b, which performs imaging in the illumination range, from each other. In the present embodiment, the entire distal-end cover 120 is transparent and the areas of the illumination window 120a and the imaging window 120b partly overlap each other.

The imaging unit 111 is provided on an imaging board 124 with a solid-state imaging device 125 formed of, for example, a CCD, which performs imaging in the range that is illuminated with the illumination light L from the illumination unit 112a, 112b, and an image forming lens 126 which includes a fixed lens 126a and a movable lens 126b, and forms the image of a subject to the solid-state imaging device 125, and executes sharp image forming with a focus adjusting unit 128 with a fixed frame 128a which secures the fixed lens 126a and a movable frame 128b, which secures the movable lens 126b. In the present invention, the imaging unit 111 is not limited to the CCD, but an imaging unit such as CMOS, may be used.

The illumination units 112a, 112b are provided on an illumination board 130 and are comprised of, for example, a light-emitting diode (LED), and a plurality of illumination units 112a, 112b (four in the present embodiment as one example) are laid out around the image forming lens 126 which constitutes the imaging unit 111. In the present invention, the illumination units 112a, 112b are not limited to the LED but other illumination units may be used as well.

The power supply unit 13 is provided on a power supply board 132 provided with an internal switch 131 and uses, for example, a button type battery as a power supply 133. While a silver oxide cell, for example, is used as the battery in the present invention, the invention is not limited to it and may use a chargeable battery, a dynamo type battery or the like.

Although one which can perform an ON operation by, for example, the oppositional action of magnets is used as the internal switch 131, the present invention is not limited to this type and other switch units can be also exemplified.

In the present embodiment, besides the individual units described above, a radio unit 142 comprising an antenna or the like for radio communication with outside is provided on a radio board 141 and communication with outside is carried out as needed.

A signal processing/control unit 143 for processing or controlling the individual units is provided on an imaging board 124 and executes various processes in the capsule endoscope 10.

The signal processing/control unit 143 includes a partial function of video signal processing, a transmission signal generating function which performs mixing of a video signal and a sync signal, affixing of an error correction code, etc., a modulation function which performs conversion to, for example, PSK, MSK, GMSK, QMSK, ASK, AM, or FM system, a power supply control function which controls power supply according to ON-OFF of a switch, driver circuits such as an LED driver circuit, a timing generator (TG) function which controls the number of imaging shots and a memory function which stores various data, such as parameters for setting the number of imaging shots, and the like, and executes various signal processes/controls.

The video signal processing function includes processes, such as image data correction (e.g., white balance (WB) correction, y correction, color processing, and AGC), and correlation double sampling or analog-digital conversion (ADC) and an auto exposure function (AE) if necessary.

Besides the radio unit 142, for example, information collecting units, such as various sensors, a chemical releasing unit which releases chemicals, a tissue collecting unit which cuts tissues in a celom and collects them, etc. may be disposed in the capsule endoscope 10 as needed.

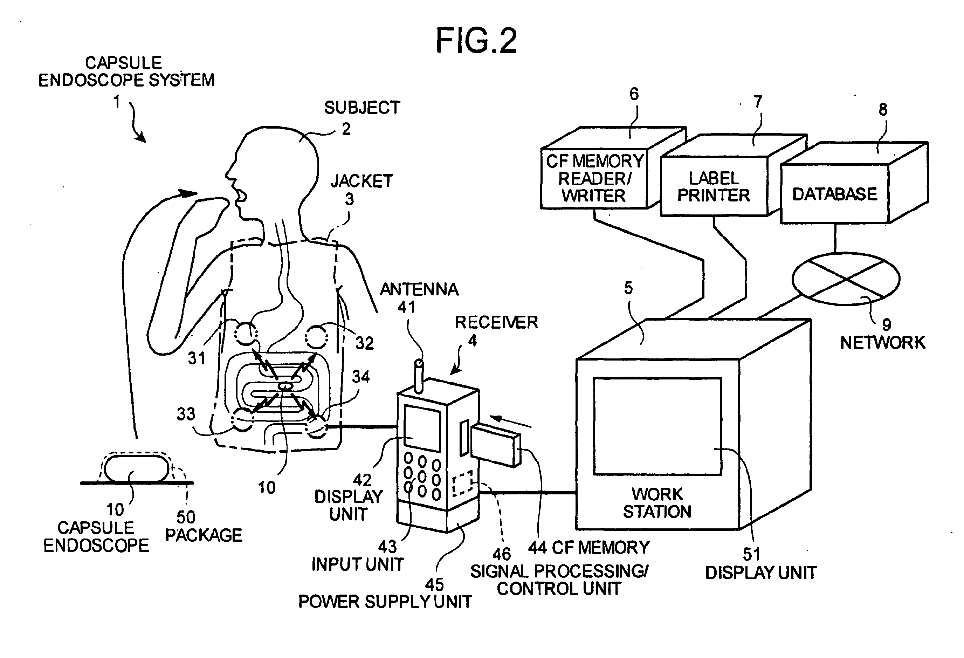

A capsule endoscope system according to the present illustrative example is described with reference to Fig. 2. Fig. 2 is a schematic diagram of a capsule endoscope system according to the embodiment. At the time of performing examination using the capsule endoscope 10, the capsule endoscope system as shown in Fig. 2 is used.

The capsule endoscope system according to the present illustrative example comprises the capsule endoscope 10 and its package 50, a jacket 3 which a patient or a subject 2 wears, a receiver 4 attachable to/detachable from the jacket 3, a work station 5, a CF (compact flash (registered trademark)) memory reader/writer 6, a label printer 7, a database 8, and a network 9, as shown in Fig. 2, for example.

The jacket 3 is provided with antennas 31, 32, 33, and 34 which catch radio waves of taken images to be sent from the radio unit 142 of the capsule endoscope 10 so that the jacket 3 can communicate with the receiver 4 wirelessly or by a cable. The number of antennas is not particularly limited to four but should be plural, so that radio waves according to positions of the capsule endoscope 10 moved can be received properly.

The receiver 4 is provided with an antenna 41 which is used when directly receiving taken images through radio waves, a display unit 42 which displays information necessary for observation (examination) and an input unit 43 which inputs information necessary for observation (examination). A CF memory 44 which stores received taken image data can be detachably attached to the receiver 4. Further, the receiver 4 is provided with a power supply unit 45 capable of supplying power even at the time of portable usage and a signal processing/control unit 46 which performs processes needed for observation (examination). As the power supply unit 45, for example, a dry cell, Li ion secondary battery, and Ni hydrogen battery can be exemplified and a chargeable type may also be used.

The work station 5 has a processing function for performing a diagnosis based on images of organs or the like in a patient, taken by the capsule endoscope 10 by a doctor or a nurse. This work station 5 has interfaces, though not shown, which connect to the receiver 4, the CF memory reader/writer 6, and the label printer 7 in a communicable manner and executes read/write of the CF memory 44, chart printing, etc.

The work station 5 has a communication function for connecting to the network 9 and stores doctor results of a patient into the database 8 via the network 9. Further, the work station 5 has a display unit 51, and receives taken image data of inside a patient from the receiver 4 and displays the images of organs or the like on the display unit 51.

As shown in Fig. 2, as the capsule endoscope 10 is taken out of the package 50 and is swallowed by the subject 2 through the mouth, prior to initiation examination, it passes through the esophagus, moves inside the celom by peristalsis of the digestive tracts and takes images inside the celom one after another.

The radio, waves of taken images are output via the radio unit 142 as needed or for the imaging results and are caught by the antennas 31, 32, 33, and 34 of the jacket 3. A signal from the antenna the intensity of whose received radio waves is high is sent to the receiver 4 outside.

In the receiver 4, taken image data received one after another is stored in the CF memory 44. The receiver 4 is not synchronized with the start of imaging of the capsule endoscope 10 and the initiation of reception and end of reception are controlled by manipulation of the input unit 43. The taken image data may be still picture data taken by plural frames per second for dynamic display or ordinary moving picture data.

When observation (examination) of the subject 2 by the capsule endoscope 10 is finished, the taken image data stored in the CF memory 44 is transferred to the work station 5 via a cable. The work station 5 memorizes the transferred taken image data in association with individual patients.

The taken image data inside the celom taken by the capsule endoscope 10 and stored in the receiver 4 in this manner is displayed by the display unit 51 of the work station 5. Accordingly, acquisition of effective data for physiological study and diagnosis of lesion can be carried out over the entire digestive tracts of a human body including the deep body portion (small intestine, etc.) which cannot be reached by an ultrasonic probe, endoscope, etc.

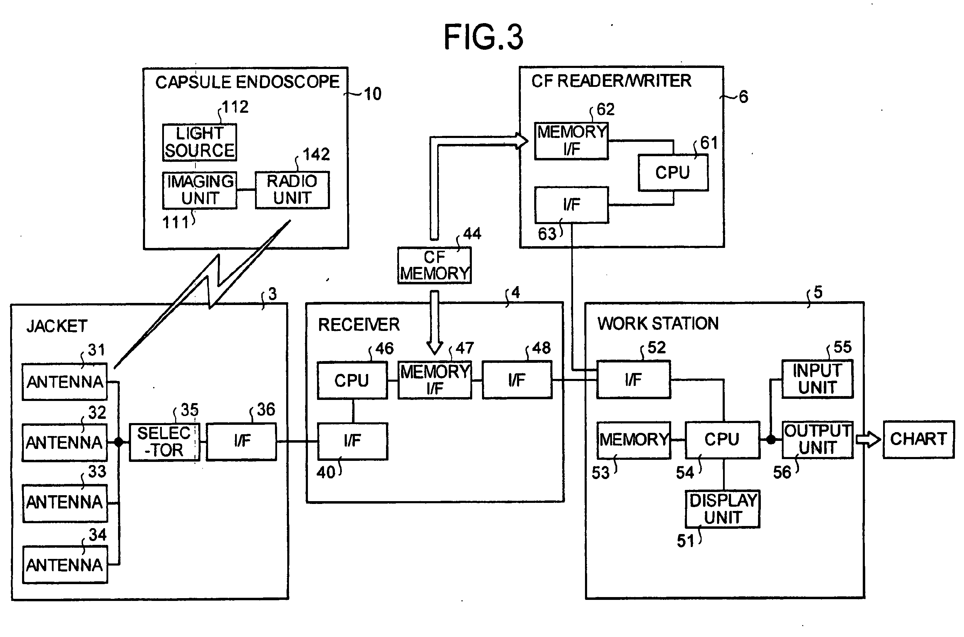

The processing system of the capsule endoscope system is described with reference to Fig. 3. Fig. 3 is a block diagram showing a structural example of the capsule endoscope system according to the embodiment.

As already explained using Fig. 1, the capsule endoscope 10 has the structure to take the image of an internal target (organs, etc.) with the imaging unit 111 from reflection of light illuminated from the illumination units 112a and 112b and send the taken image from the radio unit 142 in the form of a radio signal.

The jacket 3 has a structure such that a selector 35 is connected to the four antennas 31, 32, 33, 34, and an I/F 36 to which a cable to connect to the receiver 4 is connected to the selector 35. The jacket 3 receives radio signals sent from the capsule endoscope 10 at the four antennas 31, 32, 33, and 34, select a received signal according to the radio wave intensity by the selector 35 and is transferred to the receiver 4 via the I/F 36. The jacket 3 is not provided with a large-capacity memory and taken images received via the antennas 31, 32, 33, and 34 are transferred one after another to the receiver 4 at the subsequent stage.

The receiver 4 has, as the internal structure, an I/F 40 for communication to the I/F 36 of the jacket 3 via a cable, a CPU 46 which controls the entire receiver 4 according to a program prepared beforehand, a CF memory I/F 47 which performs data communication with the attached CF memory 44, and an I/F 48 which performs communication with the work station 5 by a cable.

To secure the state of being capable of receiving taken images from the jacket 3 at any time, the receiver 4 is always attached to the subject 2 during observation of inside a body by the capsule endoscope 10. During observation, therefore, taken images are received one after another from the jacket 3 and the received images are stored in the CF memory 44 via the CF memory I/F 47 one after another. During observation, the receiver 4 is not connected to the work station 5 and the subject 2 is not restricted in a hospital or the like and can move freely.

The CF memory reader/writer 6 has, as the internal structure, a CPU 61 which controls the entire reader/writer according to a program prepared beforehand, a CF memory I/F 62 which performs data communication with the attached CF memory 44, and an I/F 63 which performs communication with the work station 5 by a cable.

The CF memory reader/writer 6 is attached with the CF memory 44 and is connected to the work station 5 via the I/F 63, performs formatting of taken information for diagnosis according to the present embodiment with respect to the CF memory 44 or reads stored taken image data from the CF memory 44 and transfers the data to the work station 5. The taken image data here is in the form of JPEG or the like.

According to the present illustrative example, as apparent from the above, it is possible to arbitrarily select direct transfer of taken image data to the work station 5 from the receiver 4 or moving the CF memory 44 to the CF memory reader/writer 6 to transfer taken image data to the work station 5.

The work station 5 has the display unit 51 which displays images of organs, etc. according to the present embodiment, an I/F 52 which manages communication with the I/F 48 of the receiver 4 via a cable and the I/F 63 of the CF memory reader/writer 6 via a cable, a large capacity memory 53 which stores data to be handled in various processes, a CPU 54 which controls the entire work station 5 according to a program prepared beforehand, an input unit 55 which inputs various kinds of operations and an output unit 56 which is connected to the label printer 7 or the database 8 or other printers over the network 9 for performing various kinds of output processes.

When the observation period ends and the receiver 4 is connected to the work station 5 in a communicable manner, taken image data stored in the CF memory 44 is transferred from the receiver 4 to the work station 5 and stored in the memory 53. In the work station 5, taken images from the capsule endoscope 10 according to the present illustrative example , the display of an average color slider to be discussed later, the locus of the capsule endoscope 10, etc. are displayed at the time of a diagnosis. The diagnosis results are output as a chart from the printer and stored in the database 8 patient by patient.

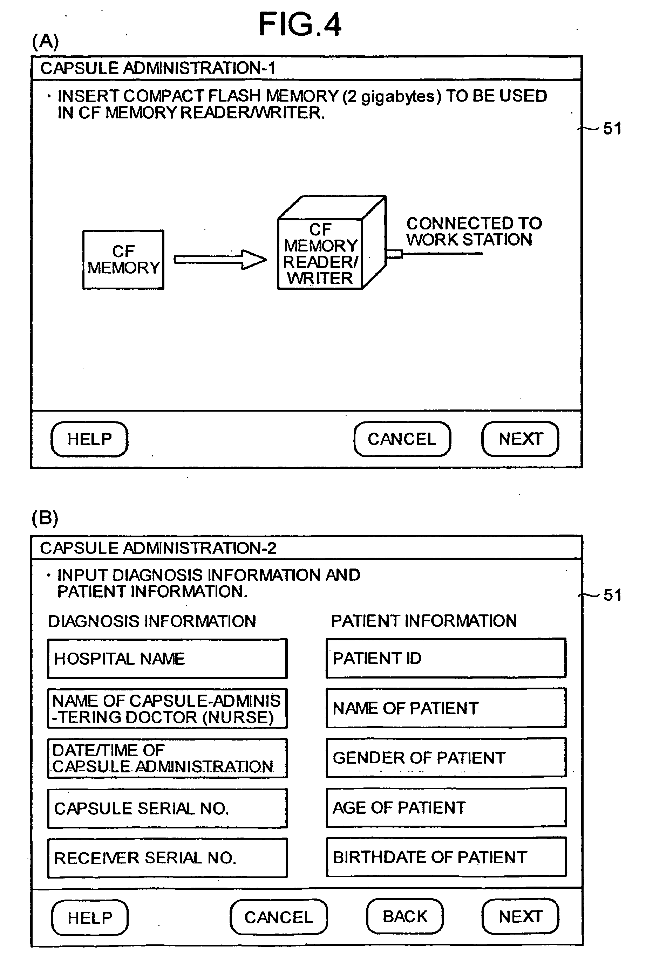

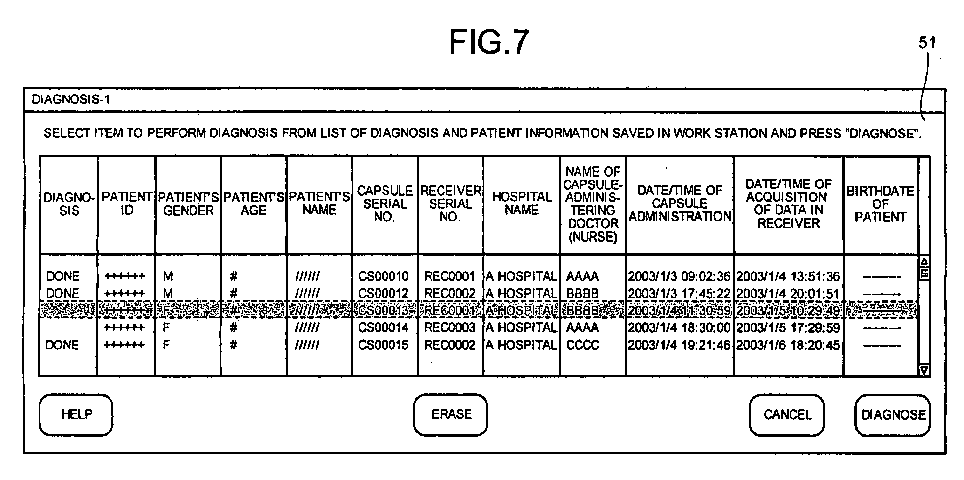

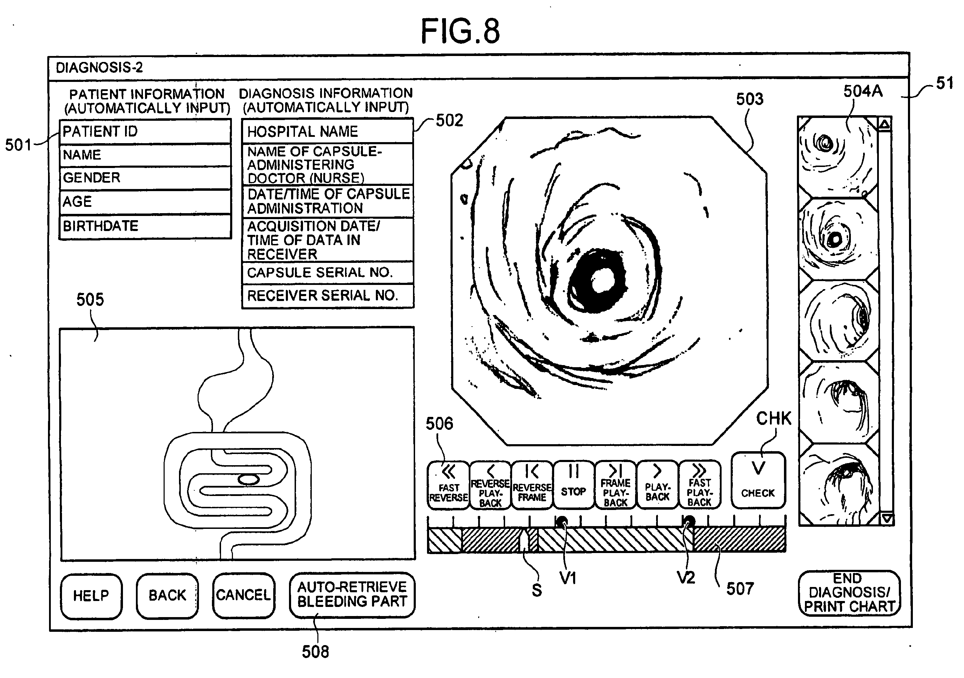

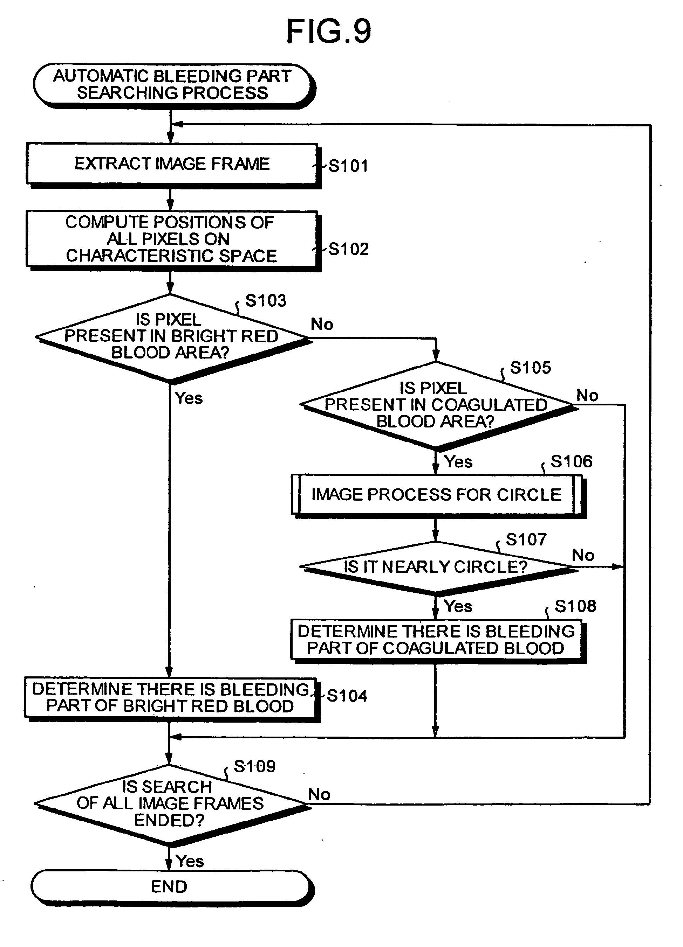

Next, specific procedures according to the present illustrative example are explained. Figs. 4, 5, and 6 show one example of screen transition associated with the observation procedures according to the present embodiment, Figs. 7 and 8 show one example of screen transition associated with the diagnosis procedures according to the present illustrative example, and Fig. 9 is a flowchart of the operation for average color bar display according to the present embodiment. A program for displaying an average color slider is directly installed from a recording medium such as CD-ROM or is downloaded from outside such as a network, then installed and stored in the memory 53 of the work station 5 as its storage scheme.

First, a doctor (or a nurse) formats the CF memory 44 using the work station 5 and the CF memory reader/writer 6. In this case, as procedures prior to observation, the CF memory 44 is inserted into the CF memory reader/writer 6 and a guidance screen prompting connection of the CF memory reader/writer 6 to the work station 5 is displayed on the display unit 51 of the work station 5 (Fig. 4(A)). When the doctor performs a menu operation for "NEXT", the process proceeds to the next guidance screen display. It is assumed that the doctor has prepared according to the guidance at this time. If the preparation is inadequate and the menu operation for "NEXT" is done in-that state, a message of non insertion of the CF memory, non-connection of the CF memory reader/writer or the like may be displayed.

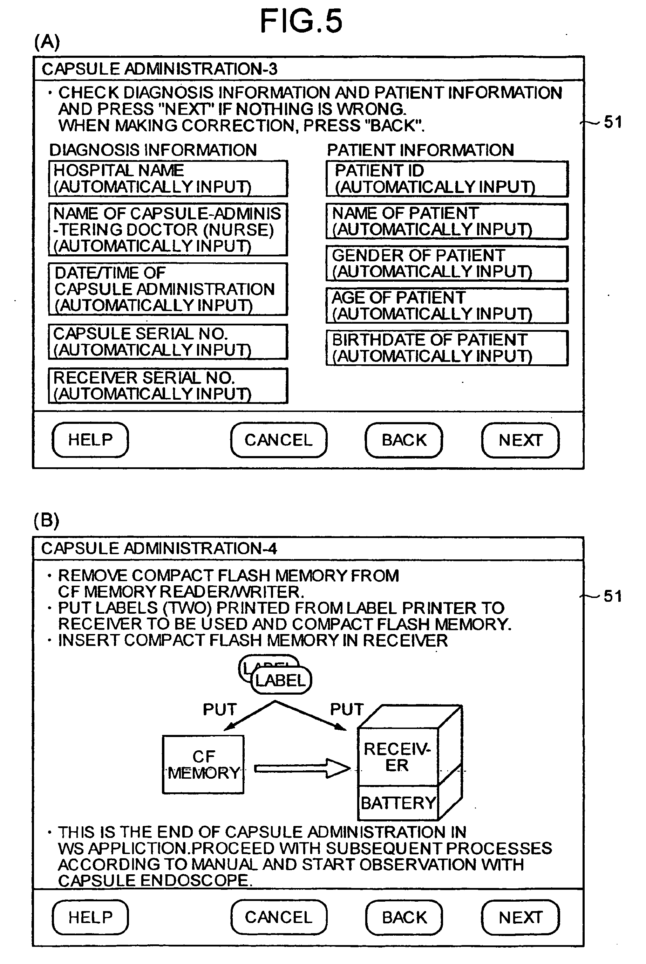

The next guidance screen displays a guidance screen prompting entry of diagnosis information and patient information (Fig. 4(B)). As the diagnosis information, there are input items of, for example, a hospital name, the name of capsule-administering doctor (nurse), the date/time of capsule administration, a capsule serial number and a receiver serial number. As the patient information, there are input items of, for example, a patient ID, the name of a patient, gender of the patient, the age of the patient and the birth date of the patient. When the input operation for various input items is completed and the menu operation for "NEXT" is done, a confirmation screen for the entered items is displayed (Fig. 5(A)). The screen may go back to the previous screen through a menu operation for "BACK".

As the next guidance screen (Fig. 5(A)) shows a confirmation of the items entered on the previous screen and the doctor further performs the menu operation for "NEXT", it is considered that nothing is wrong about the input information and the display screen goes to the next screen (Fig. 5(B)). At this time, information on the input items is written in the CF memory 44. When the menu operation for "BACK" is done, the items entered previously can be corrected.

The next guidance screen (Fig. 5(B)) shows a message of an instruction to remove the CF memory 44, an instruction to put labels having necessary ID information printed according to the input items confirmation of the items entered on the previous screen to the receiver 4 and the CF memory 44, and an instruction to insert the CF memory 44 into the receiver 4. When the doctor performs a menu operation for "COMPLETED", preparation before administration of the capsule endoscope 10 into the subject is completed.

Then, the administration of the capsule endoscope 10 into the subject 2 is completed, observation of the interior of the body is started and storage of taken image data into the CF memory 44 is started by the operation of the receiver 4. When the observation period ends and storage into the CF memory 44 is finished, the doctor receives guidance from the work station 5 again.

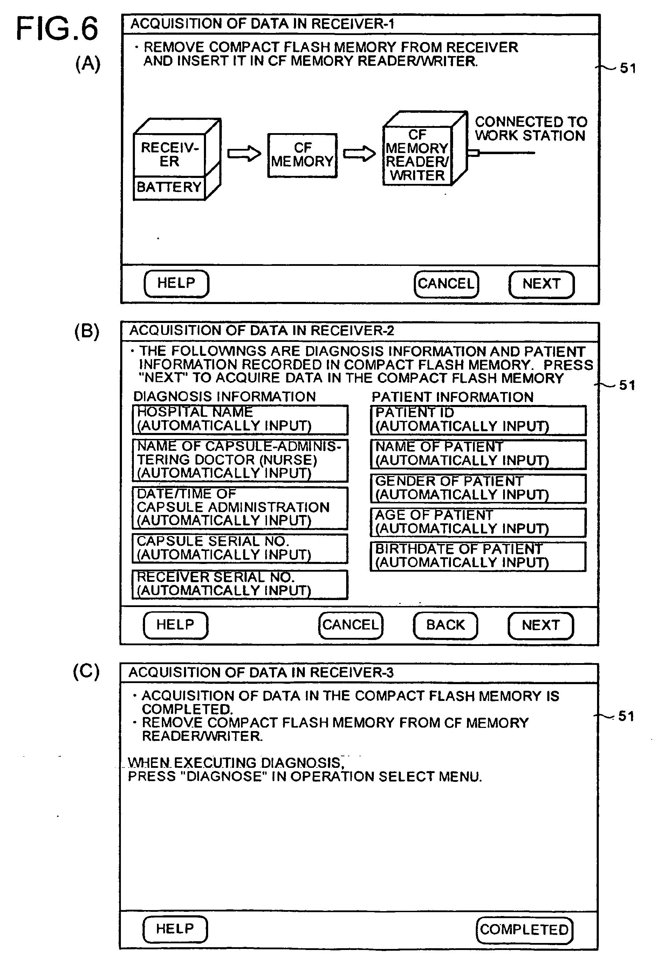

First, the CF memory 44 is removed from the receiver 4 and a guidance screen prompting insertion of the-CF-memory-reader/writer 6 is displayed (Fig. 6(A)). After preparation takes places according to the message, when the doctor performs the menu operation for "NEXT", the display screen goes to the next (Fig. 6(B)).

In the next guidance screen (Fig. 6(B)), the diagnosis information and patient information recorded in the CF memory 44 are read from the memory and displayed. The information of the displayed contents, i.e., information (taken image data, etc.) acquired through observation is acquired by the work station 5.

When the doctor performs the menu operation for "NEXT" upon completion of acquisition of the information in that manner, a process of acquiring data from the CF memory 44 is carried out. When the data acquisition process is finished, a guidance screen prompting completion of data acquisition from the CF memory 44, removal of the CF memory 44 from the CF memory reader/writer 6 and instruction for initiation of diagnosis is displayed (Fig. 6(C)). When the doctor performs the menu operation for "COMPLETED", a sequence of guidance associated with the observation procedures is completed.

In the transition of a series of screens, there are icons of CANCEL and HELP which the doctor can arbitrarily select and operate. When the CANCEL is operated, the inputs so far are initialized.

At the stage of the diagnosis process, first, a list of diagnosis information and patient information of individual patients saved in the memory 53 of the work station 5 is displayed (Fig. 7). Accordingly, the doctor can select on which patient diagnosis is to be done with, for example, a cursor. The selected state has only to be predetermined in inverted display. When a menu operation for "OBSERVATION" is done with the cursor selecting state, a patient to be diagnosed is decided. With regard to diagnosed patients, affixing "DONE" on the displayed list as shown in Fig. 7 can ensure an easy confirmation of whether a diagnosis has been made.

As a patient to be diagnosed is decided in this manner, a diagnosis procedure screen is displayed as shown in Fig. 8. This diagnosis procedure screen shows information necessary for diagnosis. 501 and 502 are respectively patient information and diagnosis information of the associated patient, and 503 is an image display field illustrating one of taken images. 504A shows a checked-image display field giving a list of taken images of interest which-have been arbitrarily checked (selected) by a doctor by operating a software-based check button CHK.

505 shows a 3D (three dimensional) position display field showing an imaging position (position inside a body) of the taken image, displayed in the image display field 503, in a 3D manner, 506 shows a playback operation field 506 for performing a playback operation for a taken image to be displayed in the image display field 503, and 507 shows an average color bar colored in time sequence with average colors according to the organs for taken images from the start point of reception by the receiver to the end point of reception. The average color bar 507 serves as a scale indicating the passing time during the observation period. The display screen further displays individual menus for "HELP", "BACK", "CANCEL", and "END DIAGNOSIS/PRINT CHART".

The average color bar 507 is average colors acquired from the individual frames of a taken image and colored in time sequence using the characteristics of colors different from one organ to another. In the average color bar 507, therefore, the average color of a taken image when the capsule endoscope 10 is moving according to regions of each organ becomes nearly uniform. Even if an image taken while movement in the same organ contains noise, nearly a uniform color for each organ can be acquired by obtaining the average color of a single screen frame by frame.

In the average color bar 507, a slider S is shown movable in the direction of the time axis. The slider S serves as an index to indicate the position of a taken image to be displayed in the image display field 503, at a position on the average color bar 507. Therefore, moving/display control of the slider S is carried out according to the operation of the playback operation field 506.

The movement of the slider S on the average color bar 507 and changing of the taken image to be displayed in the image display field 503 are synchronized. That is, a software-based FRAME PLAYBACK button, PLAYBACK button, and FAST PLAYBACK (FP) button for operations in the forward playback direction along the time-sequential direction and a software-based REVERSE FRAME PLAYBACK button, REVERSE PLAYBACK button, and FAST REVERSE PLAYBACK (FR) button for operations in the reverse playback direction along the time sequential direction are displayed and controlled. Further, a STOP button is displayed and controlled in the playback operation field 506.

When a doctor clicks the PLAYBACK button with a mouse (not shown) by operating the input unit 55, an image based on taken image data is displayed in the image display field 503 in time sequence in the forward playback direction. When the FRAME PLAYBACK button is clicked, a next image in the forward playback direction is displayed, and when the FAST PLAYBACK button is clicked, images are reproduced and displayed faster than the playback done by the PLAYBACK button in the forward playback direction. When the STOP button is clicked during playback or during fast playback, changing of the displayed image is stopped while an image at the time the clicking was made is displayed.

When the doctor clicks the REVERSE PLAYBACK button with the mouse (not shown) by operating the input unit 55, an image based on taken image data is displayed in the image display field 503 in the reverse playback direction with respect to the time-sequential direction. When the REVERSE FRAME PLAYBACK button is clicked, an image previous by one in the forward playback direction is displayed, and when the FAST REVERSE PLAYBACK button is clicked, images are reproduced and displayed faster than the playback done by the REVERSE PLAYBACK button in the reverse playback direction. When the STOP button is clicked during reverse playback or during fast reverse playback, changing of the displayed image is stopped while an image at the time the clicking was made is displayed.

When a diseased part like a bleeding part is found, or the like at the time of image playback or reverse playback in the image display field 503, a checked image distinguished from other images can be extracted at the doctor's discretion. When such checking is desired, the doctor operates the check button CHK. The checked image is additionally displayed as a thumbnail image in the checked-image display field 504A. Due to the restriction of the display area, the checked-image display field 504A can display up to a predetermined number of images. In the present embodiment, as shown in Fig. 8, for example, up to five images can be displayed and for other checked images, display images are switched by scrolling.

As the average color bar 507 is segmented by the average colors according to the types of the organs, the doctor can intuitively and quickly move the display image to the position of the taken image associated with the desired organ referring to the average color bar 507. At this time, the slider S of the average color bar 507 is moved by using the mouse (not shown). As the slider S is operated to move on the average color bar 507, a process of sequentially changing the image to the one at the position indicated by the slider S following the movement is executed in the image display field 503.

In the present illustrative example, when the doctor finds a bleeding part from the display image, a flag as a bleeding part can be affixed to each taken image. In this case, though not shown, a sub menu is displayed with the current state displayed in the image display field 503 to manually set the flag of the bleeding part. Accordingly, display can be made in association with the positions on the average color bar 507, such as bleeding parts V1, V2, as shown in Fig. 8, for example.

A bleeding part can be automatically extracted through image processing, in which case an AUTO-RETRIEVE BLEEDING PART button 508 is operated. The operation of the AUTO-RETRIEVE BLEEDING PART button 508 may be done for the image currently displayed in the image display field 503 or for all the images. When a bleeding part is found in automatic retrieval, a flag is put in association with each image as done in the case of manual operation, and when displaying an image, it is desirable to display the bleeding parts V1 and V2 corresponding to the flag.

The diagnosis by a doctor can be terminated by a menu operation for "END DIAGNOSIS/PRINT CHART". The diagnosis results are made into a chart and printed through a printer (not shown) from the work station 5 or via the database 8.

Referring to Figs. 9 to 12, the procedures of an automatic bleeding part searching process which is carried out by the CPU 54 are described below. Referring to Fig. 9, the CPU 54 acquires one image frame from the memory 53 first (step S101), and computes the positions of all the pixels in the acquired image frame on the characteristic space (step S102).

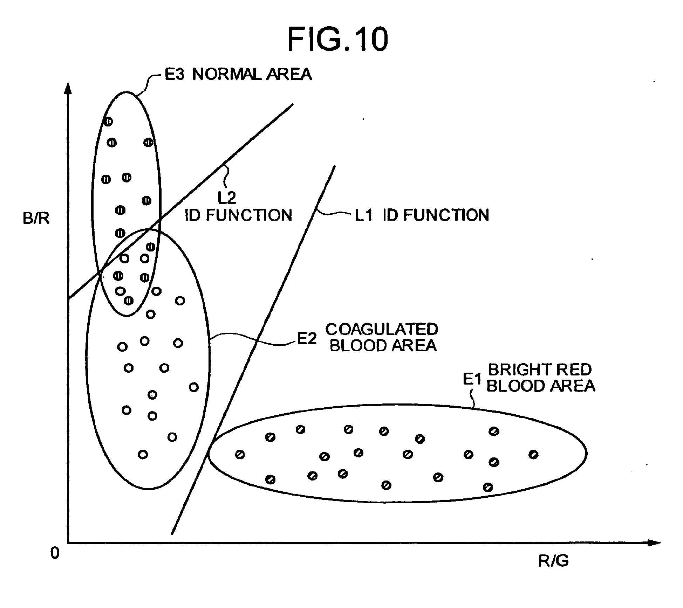

As shown in Fig. 10, the characteristic space is color space with R/G (red element/green element) taken on a horizontal axis and B/R (blue element/red elements) taken on a vertical axis. When there is bleeding, it is possible to determine, based on the distribution in the characteristic space whether it is bright red bleeding, coagulated blood, or normal bleeding. As shown in Fig. 10, a bright red blood area E1, a coagulated blood area E2, and a normal blood area E3 are formed in the characteristic space, and the bright red blood area E1 and the coagulated blood area E2 are distinguished from each other by an ID function L1 while the coagulated blood area E2 and the normal blood area E3 are distinguished from each other by an ID function L2. The coagulated blood area E2 and the normal blood area E3 partially overlap each other, and the ID function L2 includes a part of the normal blood area E3. When a pixel of interest has the color of an area with greater R/G than the ID function L1, it is determined as bright red blood. When a pixel of interest has the color of an area sandwiched between the ID functions L1 and L2, it is determined as at least coagulated blood. When a pixel of interest has the color of an area with greater B/R than the ID function L2, it is determined as normal bleeding.

Referring to Fig. 9, after the positions of all the pixels on the characteristic space are computed (step S102), the CPU 54 determines whether those pixels contain a pixel included in the bleeding area E1 (step S103). This determination is made by using the ID function L1 and checking if the computed position of each pixel on the characteristic space lies on the right-hand side to the ID function L1 in Fig. 10. When there is a pixel in the bright red blood area E1 (YES at step S103), it is determined that there is a bright red blood part (step S104), and the flow goes to step S109.

When there is no pixel in the bright red blood area E1 (NO at step S103), on the other hand, it is further determined whether there is a pixel in the coagulated blood area E2 (step S105). This determination is made by checking if a pixel is positioned in the area sandwiched by the ID functions L1 and L2. When there is a pixel in the coagulated blood area E2 (YES at step S105), image processing for a circle is performed to analyze whether the bleeding part which contains those pixels is nearly a circle (step S106). This circular image processing is performed because when the bleeding part is coagulated blood, it becomes nearly circular. When the bleeding part is bright red blood, the periphery of the bleeding part becomes wavy and does not become nearly circular.

Thereafter, it is determined based on the results of the circular image processing whether the bleeding part is nearly a circle (step S107). When the bleeding part is nearly a circle (YES at step S107), it is determined that the bleeding part is a coagulated blood part (step S108), and the flow goes to step S109. When there is no pixel in the coagulated blood area (NO at step S105) and when the bleeding part is not nearly a circle (NO at step S107), it is determined that the bleeding part is coagulated blood, normal bleeding or no bleeding, and the flow goes to step S109.

At step S109, it is determined whether the bleeding part searching has been completed for entire image frames. When there is an image frame which should undergo the search, the flow goes to step S101 to repeat the processes. When there is no image frame which should undergo the search, the routine is terminated. When it is determined that there is a bright red blood part or a coagulated blood bleeding part, a flag indicating the determination is affixed to the associated image frame.

Although the determination processes at the step S103 and the step S105 do not refer to the number of pixels, there should be one pixel or more in an image frame. Due to the possibility that a pixel being noise, however, it is preferable to determine whether there are a predetermined number of pixels.

While the ID functions L1 and L2 are used in the determination processes at the step S103 and the step S105, the determination is not restrictive and may be made without using the ID functions L1 and L2 but by checking if each pixel is positioned in the bright red blood area E1, the coagulated blood area E2, or the normal blood area E3.

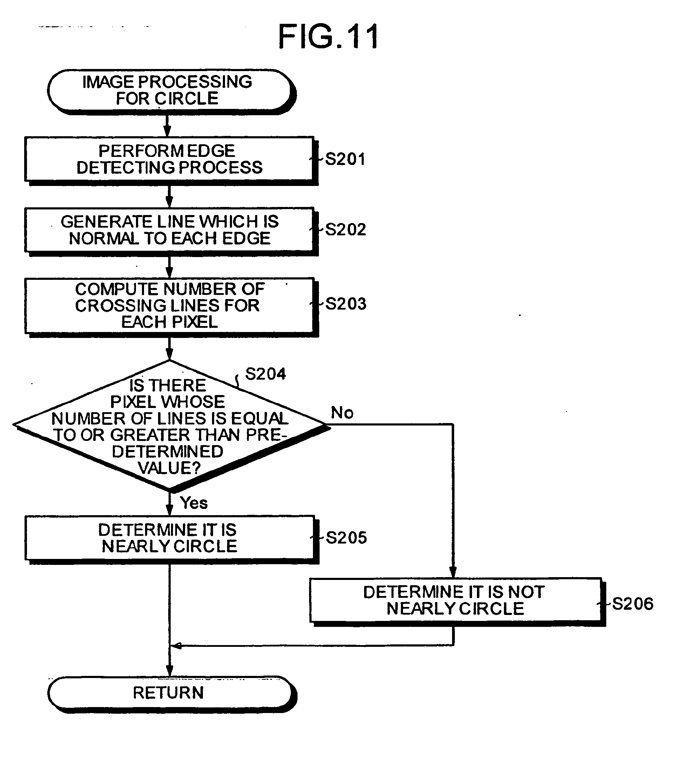

The following describes the circular image processing performed at the step S106. Fig. 11 is a detailed flowchart of procedures of the circular image processing. As shown in Fig. 11, the CPU 54 first performs edge detection for each pixel by the Sobel method or the like (step S201). The CPU 54 then generates lines which are normal to detected individual edges (step S202). Further, for each pixel, the CPU 54 computes how many lines cross the pixel (step S203).

It is then determined whether the computed-number of lines is equal to or greater than a predetermined value (step S204). When the number of lines is equal to or greater than the predetermined value (YES at step S204), the bleeding part is determined as nearly a circle (step S205), and the flow returns to step S106. When the number of lines is not equal to or greater than the predetermined value (NO at step S204), the bleeding part is determined as not nearly a circle (step S206), and the flow returns to step S106.

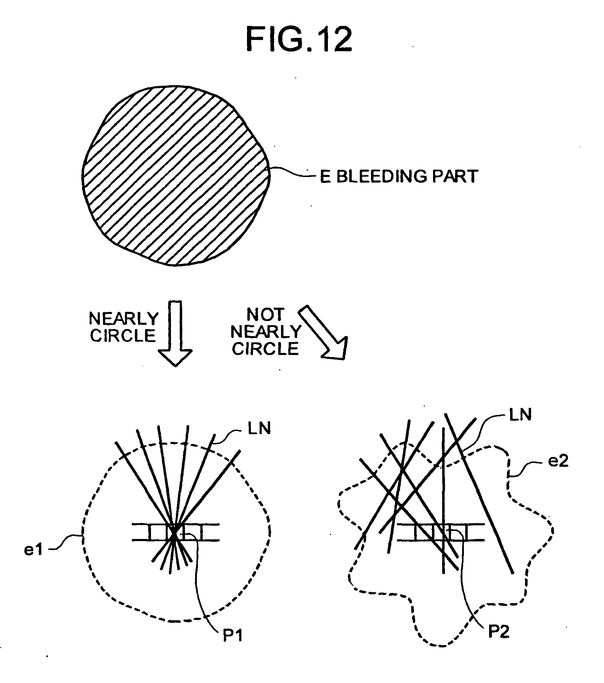

For example, as shown in Fig. 12, when an edge e1, e2 corresponding to the bleeding part E is detected in the edge detection process, lines LN, which are normal to the edge of each pixel, are generated, and it is determined whether the pixel is a pixel P1 for which the number of intersections of the lines LN is equal to or greater than a predetermined value, or a pixel P2 for which the number of intersections of the lines LN is less than the predetermined value. When there is the pixel P1 for which the number of intersections is equal to or greater than the predetermined value, the edge e1 is determined as nearly a circle.

Although the determination at the step S204 is made by checking if there is a pixel for which the number of lines is equal to or greater than a predetermined value, the determination is not restrictive. For example, it may be further determined whether there are a predetermined number of, or a greater number of, pixels for each of which the number of lines is equal to or greater than a predetermined value.

As the CPU 54 performs the automatic bleeding part searching process and marks a bleeding part via a flag, a search for a bleeding part from a vast amount of image information, which is troublesome and takes a considerable time when it is done by a doctor or a nurse, can be executed easily and quickly. This reduces possible overlooking of bleeding parts and allows a doctor or a nurse to concentrate on the examination of the condition of a bleeding part.

Although the automatic bleeding part searching process is associated with a bleeding part, it is not limited to this particular type but can be adapted to other types of images to be searched. In addition, the automatic bleeding part searching process shown in Fig. 9 may not perform the process of detecting a coagulated blood area (steps S105 to S108), and may make a determination only on whether there is a bright red blood area. In other words, although the automatic bleeding part searching process searches for a bright red blood part and a coagulated blood part, the process may search only for a bright red blood part. Although the automatic bleeding part searching process does not make a determination on a normal bleeding part, it may be designed to detect a bleeding part including such a normal bleeding part.

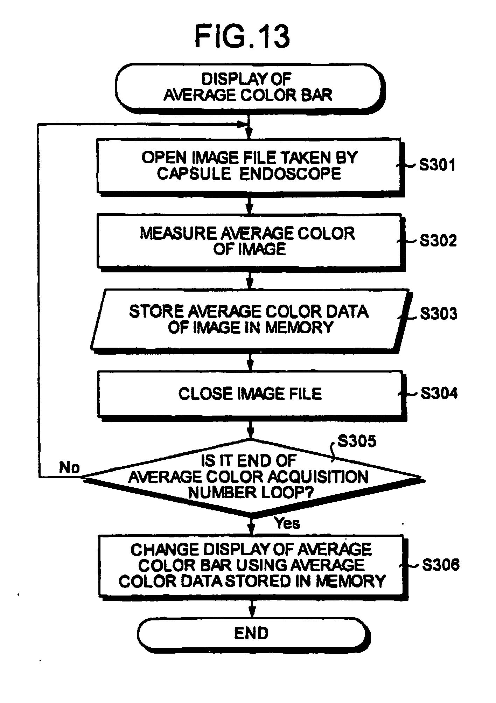

In the display of the average color bar 507, a process is executed as shown in Fig. 13. That is, when a patient to be diagnosed is decided from a list shown in Fig. 7, a file of imaging information corresponding to that patient is designed. Then, one frame of image files is read from the memory 53 and opened (step S301), and the average color of the taken images frame by frame is measured (step S302).

When the average color is measured and average color data is acquired, the average color data for the first frame is stored in the memory 53 (step S303). Then, a processed image file is closed (step S304) and an image file located next in time sequence is read out and opened, and a similar process is repeatedly executed thereafter (NO route of step S305).

When the average colors for all the imaging information of the patient to be diagnosed are obtained (step S305), the average color bar 507 is displayed and controlled as shown in Fig. 8 using the average color data stored in the memory 53 (step S306). In this manner, the display of the average color bar 507 is completed. At this time, the initial position of the slider S is the left end (start position) of the average color bar 507 but is not restrictive.

Because the amount of the imaging information including taken image data is huge, it is unnecessary to open all the image files and acquire the average colors for all the frames, and the average color may be acquired while efficiently thinning several frames. Although the acquired average color itself is displayed on the average color bar 507 in the present embodiment, it is not restrictive and a color corresponding to this average color has only to be displayed on the average color bar 507.

According to the present illustrative example, as described above, a scale indicating the overall imaging period of input image data taken in time sequence by the capsule endoscope (in vivo imaging device) is displayed, a movable slider is shown on the scale, an image at the imaging time corresponding to the position of the slider is displayed in response to the movement of the slider on the scale, and a color corresponding to average color information for one screen of input image data is displayed at the time-associated position on the scale, so that distinguishing coloring is carried out according to the taken part and an organ in the body can easily be determined from the distinguished colors. Accordingly, the ability to retrieve the image is improved and it is possible to easily recognize the organ depicted in each image.

Although the position of an organ is identified using the average colors arranged on the average color bar as an index in the embodiment described above, the present invention is not limited to this type and an additional function of displaying the name of an organ in association with the average color may be provided as in a modification to be discussed below. As the modification to be discussed below is the same in the structure and functions described above, only what is added is discussed.

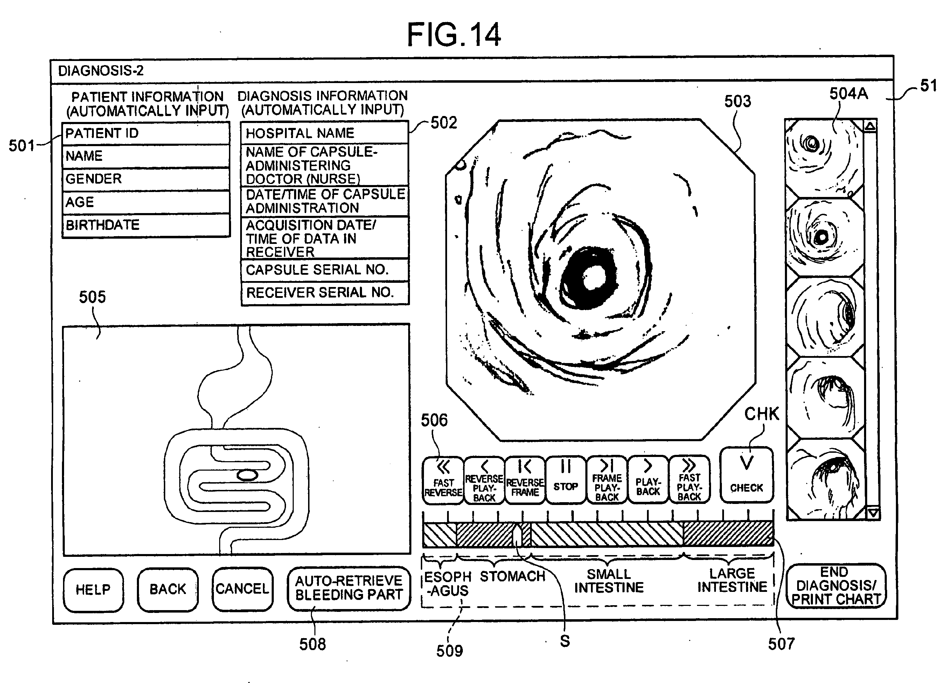

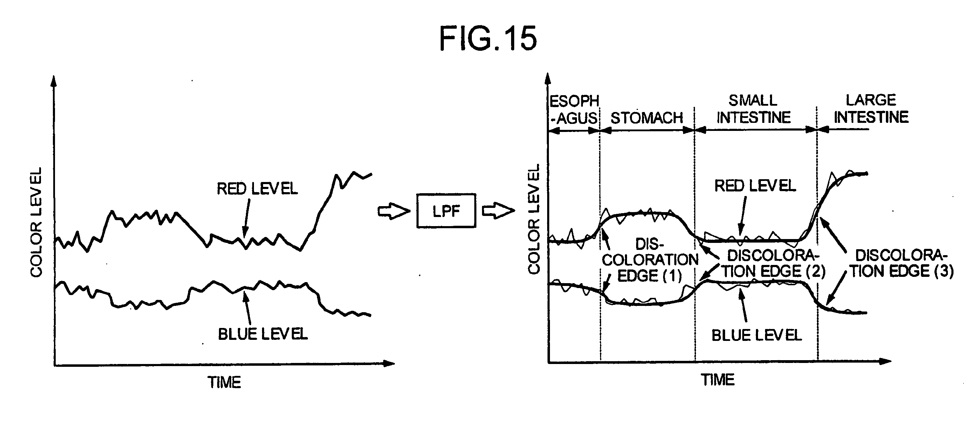

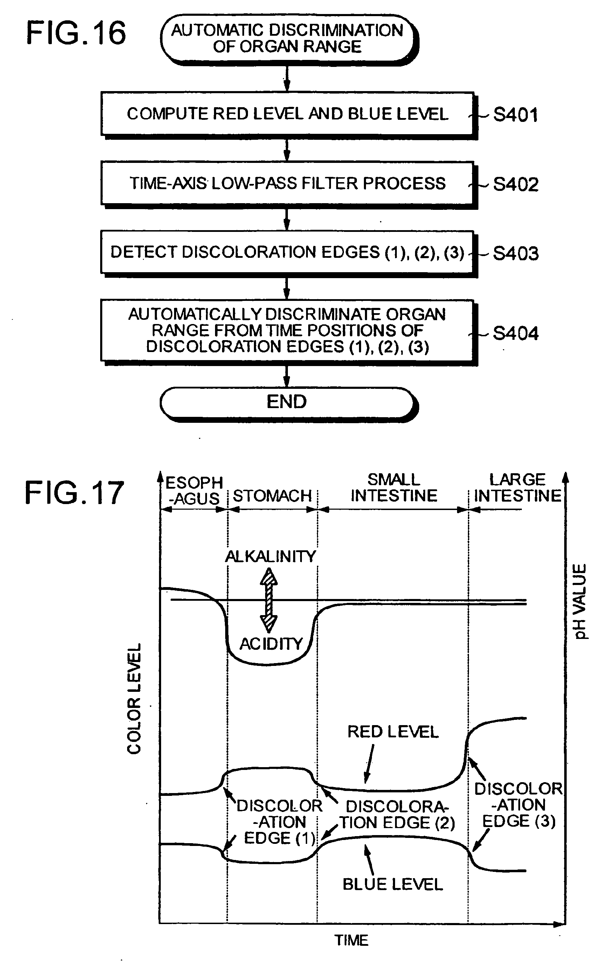

Fig. 14 shows an example of a display screen associated with a diagnosis process according to a modification of the illustrative example, Fig. 15 shows the principle of automatic discrimination of organ names according to the modification of the illustrative example, and Fig. 16 is a flowchart of the procedures of discriminating the organ names according to the modification of the illustrative example.

In Fig. 14, the organ names are displayed in association with each average color on the average color bar 507. Average colors are lined on the average color bar 507 in the order of the esophagus, the stomach, the small intestine, and the large intestine in the order of imaging done in a body by the capsule endoscope 10 in time sequence. Therefore, the average color bar 507 shows organ names 509 in the order of the esophagus, the stomach, the small intestine, and the large intestine in association with the average colors of the individual organs.

At the time of automatic discrimination of organ names, it is the automatic discrimination in the ranges of organs. The level of red and the level of blue for individual taken images at elapsed times have the characteristics as shown in Fig. 15. As an actual image contains a noise component, it is subjected to a low-pass filter (LPF) process in the direction of the time axis with respect to the levels of red and blue that have the characteristics to remove noises. Then, edge portions (discoloration edges) the levels of red and blue in the direction of the time axis after the LPF process commonly have are extracted.

In the example in Fig. 15, there are three discoloration edges, N1, N2, and N3, extracted in the above manner. Therefore, automatic discrimination is done such that from the positions of the discoloration edges N1, N2, and N3 in the direction of the time axis, the first discoloration edge N1 is a transitional portion from the esophagus to the stomach, N2 is a transitional portion from the stomach to the small intestine and N3 is a transitional portion from the small intestine to the large intestine. At this time, the order of the organ names is based on the layout of the organs to be taken by the capsule endoscope 10 in the direction of the time axis.

As the processing based on the principle described above, first, the red level and blue level are computed (step S401), the LPF process in the direction of the time axis is performed on the red level and blue level (step S402) and the discoloration edges N1, N2, and N3 are detected (step S403). Then, automatic discrimination of the ranges of the organs is carried out from the time-associated positions of the discoloration edges N1, N2, and N3 and the organ names are displayed in association with the individual average colors on the average color bar 507 (step S404).

In the above manner, a scale indicating the overall imaging period of input image data taken in time sequence by the capsule endoscope is displayed, a movable slider is shown on the scale, an image at the imaging time corresponding to the position of the slider is displayed in response to the movement of the slider on the scale, and organs are discriminated based on color information for one screen of input image data and organ names are displayed in association with the scale, so that organs in the body can easily be determined from the displayed organ names. This also improves the ability to retrieve images and makes it possible to easily recognize the organ depicted in each image.

Although the ranges of the organs on the average color bar are automatically discriminated from the discoloration edges in the modification described above, the present invention is not limited to this type and a pH sensor may be provided in the capsule endoscope 10 so that the ranges of the organs are specified more accurately using the measured pH values. In this case, the pH values are measured by the pH sensor during the observation period and like taken images, the pH values are measured in time sequence and are stored in the receiver 4. At that time, the taken images and pH values are recorded in association with each other, such as coexisting in each frame (image file).

Fig. 17 shows an example of application of the modification shown in Fig. 15. In the automatic discrimination with pH values added, as shown in Fig. 17, using the fact that the stomach is in an acidic state, an acidic part is compared with the discoloration edges N1 and N3 to discriminate the stomach part, thereby further increasing the discrimination precision.

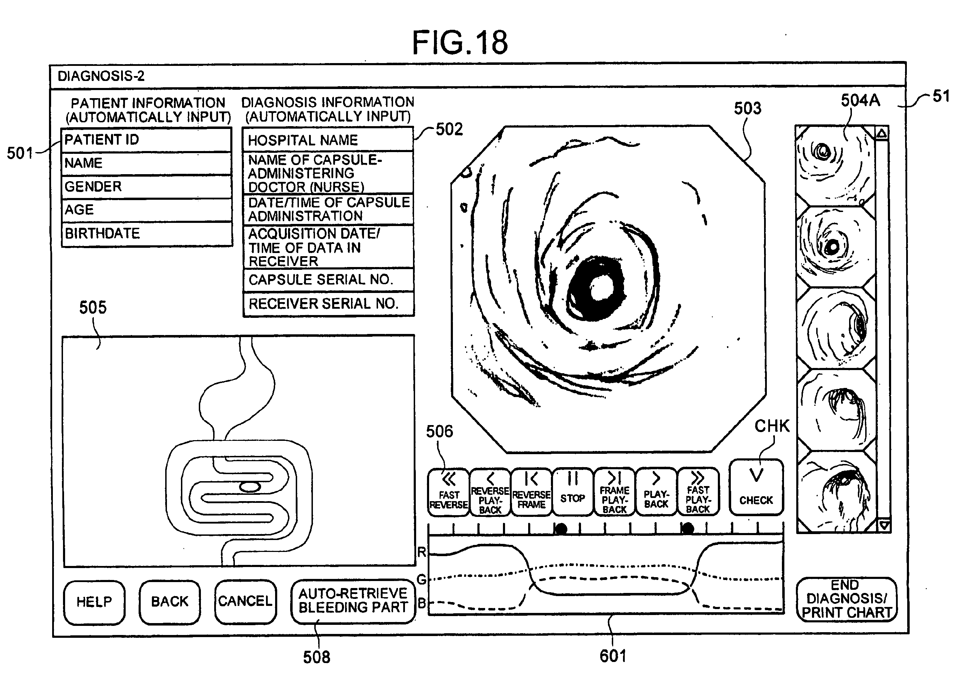

A display area 601 which shows a change in color element may be provided as shown in Fig. 18, in place of the average color bar 507. In this case, a time-sequential change in each of the average color elements (R, G, B) for each image frame is shown directly. That is, numerical parameters as color elements extracted from an image frame are transformed into visible information and shown serially in a time-sequential order. The color of the esophagus is whitish blue, the color of the stomach is red, the color of the small intestine is yellow, and the color of the large intestine is orange. As those color change, the individual color elements also change. It is possible to not only visualize a change in each color element but also specify a taken part. In this case, only one color element, for example, R, may be shown. R, G, and B of each image frame may be average values for all the pixels, or may be average values for a specific pixel, or may be average values for pixels after thinning. In other words, color element values which represent each image frame should be acquired.

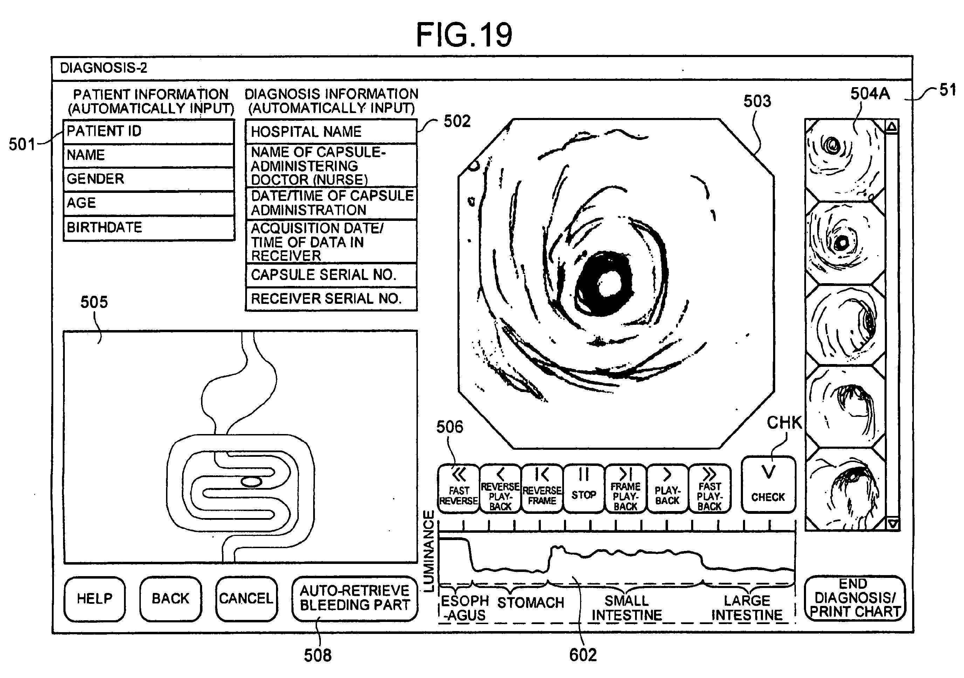

A display area 602 which shows a change in luminance may be provided as shown in Fig. 19, in place of the display of a change in each color element. The luminance Y is predetermined by Y=0.299R+0.587G+0.114B, and the luminance of each image frame can be acquired from individual color elements. Specifically, color elements extracted from an image frame are converted to numerical parameters of luminance, which are transformed into visible information to be time-sequentially and serially shown. Fig. 19 depicts organ sites according to a time-sequential change in luminance. The organ sites may be discriminated based on a change in the value of luminance or may be discriminated based on the color information or pH value.

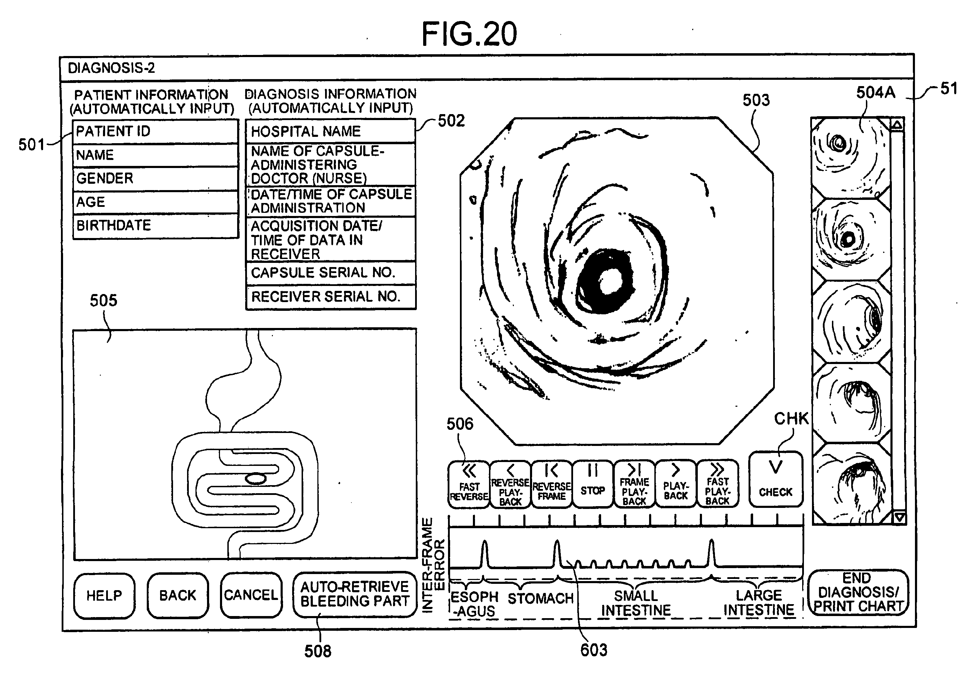

Further, a display area 603 which shows a change in inter-frame error which is a relative error among individual image frames may be provided as shown in Fig. 20, instead of the display of a change in luminance. In this case, a large inter-frame error occurs at the transition from the esophagus to the stomach, and there is a peak at the position where the change is large. The boundary between individual organ sites can be identified by directly presenting the change that produces the peak. Fig. 20 shows internal organs according to a change in time-sequential inter-frame error. The fine peaks at the small intestine in Fig. 20 are originated from the peristalsis of the small intestine.

As shown in Figs. 18 to 20, each organ site can be specified by directly displaying a time-sequential change in each color element, luminance and also inter-frame error. The organ sites may be discriminated based on the inter-frame error or may be discriminated based on the color information or pH value.

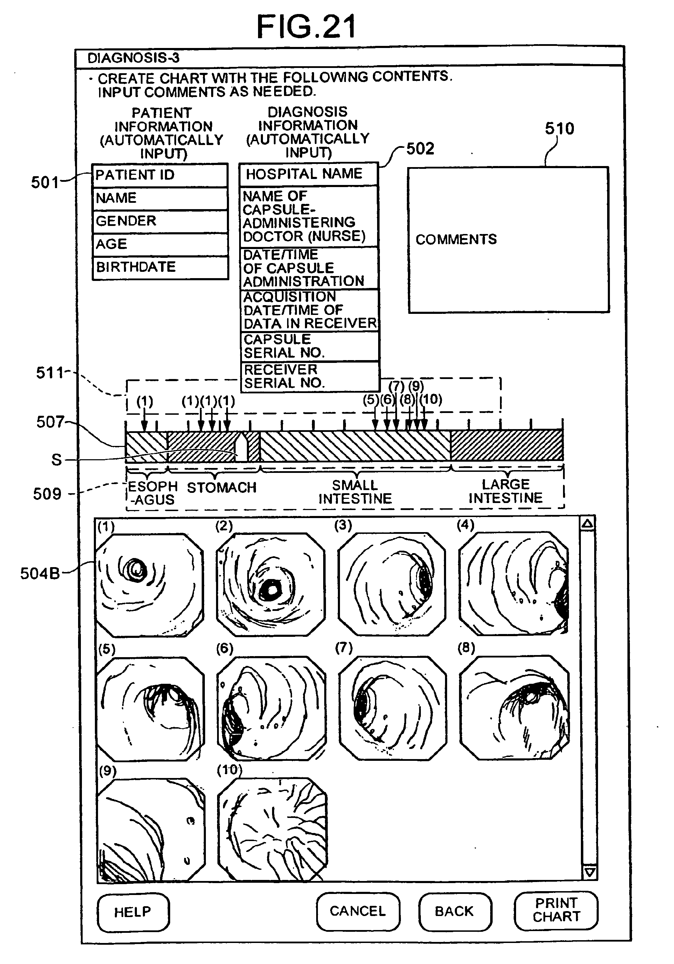

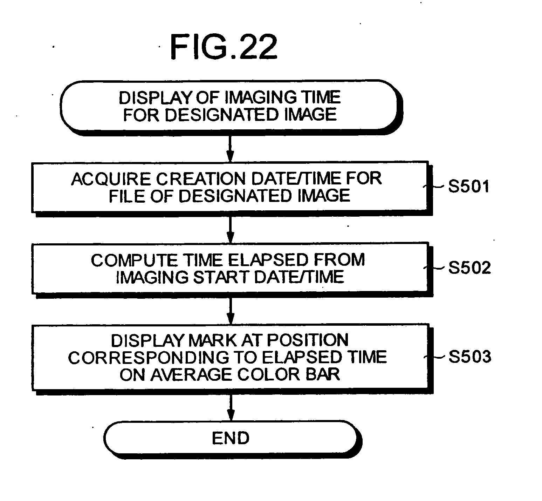

Chart creation according to the above embodiment is explained next. Fig. 21 shows one example of screen transition associated with the diagnosis procedures according to the present illustrative example, and Fig. 22 is a flowchart of an operation for displaying the imaging time of a designated image according to the illustrative example. While a diagnosis by a doctor can be terminated through the menu operation for "END DIAGNOSIS/PRINT CHART", further transition to the chart creating procedures can be made.

When the process is shifted from the display screen in Fig. 8 to the display screen in Fig. 21, comments of a doctor are entered and a mark indicating to which elapsed time on the average color bar 507 each checked image corresponds is displayed.

That is, in Fig. 21, 504B indicates a checked-image display field, set larger than the checked-image display field 504A and provided at the lower portion of the screen. -As a difference from the checked-image display field 504A, numbers C1 to C10 are predetermined to individual taken images and displayed. The checked-image display field 504B has the same function as the checked-image display field 504A.

510 is a comment input field where opinions (comments) of a doctor are input and displayed. The results of a diagnosis by a doctor are input as comments in the comment input field 510. 511 indicates an imaging time display mark that is displayed, as a mark on the average color bar 505, indicating which taken image at which elapsed time each checked image to be displayed in the checked-image display field 504B is. As the imaging time display mark, a downward arrow as an index indicating the imaging time for a checked image and the aforementioned number predetermined to a checked image as relative display indicating the correlation with the checked image to show the correlation with the checked image are displayed on the average color bar 505.

Fig. 21 shows ten checked images. In this example, average colors are distinguished on the average color bar 507 in the order of the esophagus, the stomach, the small intestine, and the large intestine. As apparent from the ranges of the organs of the organ names 509, therefore, a mark C1 for a checked image is present in the range of the esophagus, and marks C2, C3, and C4 for a checked image are present in the range of the stomach. Further, marks C5, C6, C7, C8, C9, and C10 for checked images are present in the range of the small intestine.

Therefore, the presence of images checked by a doctor are identified in the esophagus, the stomach, and the small intestine from the example in Fig. 21, and marks are displayed in association with the times at which the individual checked images have been taken, so that the doctor can easily confirm at which parts of the organs the checked images have been taken. Although the imaging time display mark is displayed on the average color bar 505 showing the organ names in Fig. 21, it may be displayed on the average color bar that does not show the organ names as in Fig. 8. Although a correlation indication (number) indicating the correlation with a checked image is displayed as the imaging time display mark in Fig. 21, it may be an index (downward arrow) indicating the position of the imaging time.

The process for the above mark display is described with reference to Fig. 22. In the imaging time display of a checked image or a designated image, first, the date/time of creating a file of the designated image is acquired from the memory 53 (step S501), and the time elapsed since the date/time of the initiation of imaging is computed (step S502). Then, a mark display as shown in Fig. 21 is controlled on the scale of the average color bar 507 at the position corresponding to the elapsed time on the average color bar 507 (step S503). Thereafter, when chart printing is manipulated, outputting for the chart printing is executed.

According to the present illustrative example, as described above, a scale indicating the overall imaging period of input image data taken in time sequence by the capsule endoscope (in-vivo imaging device) is displayed, a color corresponding to average color information for one screen of input image data is displayed at a time-associated position on the scale, an image corresponding to the input image data is displayed, and an index indicating a position corresponding to an imaging time of a designated image is displayed, so that it is possible to visually and easily recognize how many and in which time band designated images are present. As organs can easily be determined from the colors distinguished from one taken part from another one, it is possible to easily recognize which part of which organ has more designated images.

Further, a scale indicating the overall imaging period of input image data taken in time sequence by the capsule endoscope is displayed, organs are discriminated based on color information of one screen of input image data, the names of the discriminated organ are displayed in association with the scale, images corresponding to the input image data are displayed and an index indicating the position corresponding to the imaging time of the designated image is displayed on the scale, so that organs in the body can easily be determined from the displayed organ names. This also makes it possible to easily recognize which part of which organ has more designated images.

According to the present invention, distinguishing coloring is executed according to the taken part and organs in a body can easily be determined from the distinguished colors. This can improve the ability to retrieve images and ensure easy recognition of the image of which intestine the display image shows. Further, the automatic search for a bleeding part reduces the burden on a doctor or a nurse.

### INDUSTRIAL APPLICABILITY

As apparent from the above, an image display apparatus, an image display method, and an image display program according to the invention are suitable for a wireless subject internal information acquisition system which acquires an internal image of a subject using a subject probing apparatus, such as a capsule endoscope, and is suitable for use in a capsule endoscope system.

## Claims

1. An image display apparatus comprising:
an input unit (52) configured to input image data taken in time sequence by an in-vivo imaging device (10);
a scale display control unit configured to control to display a scale (507) indicating an overall imaging period of the image data taken in time sequence and input by the input unit (52) and to display a movable slider (S) on the scale (507); and
an image display control unit configured to control to display on a display unit (51) an image at an imaging time corresponding to a position of the slider in response to a movement of the slider (S) on the scale (507),
**characterized by** comprising
a color information detecting unit configured to detect color information of a screen of the image data input by the input unit, and including an average color detecting unit configured to detect an average color from the color information;
a color display control unit configured to control to display a color corresponding to the average color detected by the average color detecting unit at a position on the scale (507) corresponding to time;
an organ discriminating unit configured to discriminate an organ based on the color information detected by the color information detecting unit; and
an organ name display control unit configured to control to display a name (509) of the organ discriminated by the organ discriminating unit in association with the scale (507) so that the name (509) of the organ is displayed in association with the average color of the organ.

2. The image display apparatus according to claim 1, wherein the organ discriminating unit is configured to discriminate the organ based on information on increase and decrease of color elements constituting the color information.

3. The image display apparatus according to claim 1, wherein the organ discriminating unit is configured to discriminate the organ further based on in-vivo information acquired in accordance with the image data.

4. An image display method comprising:
an inputting step for inputting image data taken in time sequence by an in-vivo imaging device (10);
a scale display control step for controlling to display a scale (507) indicating an overall imaging period of the image data taken in time sequence and input in the inputting step and to display a movable slider (S) on the scale (507);
an image display control step for controlling to display on a display unit (51) an image at an imaging time corresponding to a position of the slider (S) in response to a movement of the slider (S) on the scale (507);
a color information detecting step for detecting color information of a screen of the image data input in the inputting step, wherein the color information detecting step includes an average color detecting step for detecting an average color from the color information;
a color display control step for controlling to display a color corresponding to the average detected in the average color detecting step at a position on the scale (507) corresponding to time;
an organ discriminating step for discriminating an organ based on the color information detected in the color information detecting step; and
an organ name display control step for controlling to display a name (509) of the organ discriminated in the organ discriminating step in association with the scale (507) so that the name (509) of the organ is displayed in association with the average color of the organ.

5. The image display method according to claim 4, wherein the organ discriminating step includes discriminating the organ based on information on increase and decrease of color elements constituting the color information.

6. The image display method according to claim 4, wherein the organ discriminating step includes discriminating the organ further based on in-vivo information acquired in accordance with the image data.

7. An image display program making a computer execute:
an inputting step for inputting image data taken in time sequence by an in-vivo imaging device (10);
a scale display control step for controlling to display a scale (507) indicating an overall imaging period of the image data taken in time sequence and input in the inputting step and to display a movable slider (S) on the scale (507);
an image display control step for controlling to display on a display unit (51) an image at an imaging time corresponding to a position of the slider (S) in response to a movement of the slider (S) on the scale (507);
a color information detecting step for detecting color information of a screen of the image data input in the inputting step, wherein the color information detecting step includes an average color detecting step for detecting an average color from the color information;
a color display control step for controlling to display a color corresponding to the average color detected in the average color detecting step at a position on the scale (507) corresponding to time;
an organ discriminating step for discriminating an organ based on the color information detected in the color information detecting step; and
an organ name display control step for controlling to display a name (509) of the organ discriminated in the organ discriminating step in association with the scale (507) so that the name (509) of the organ is displayed in association with the average color of the organ.

8. The image display program according to claim 7, wherein the organ discriminating step includes discriminating the organ based on information on increase and decrease of color elements constituting the color information.

9. The image display program according to claim 7, wherein the organ discriminating step includes discriminating the organ further based on in-vivo information acquired in accordance with the image data.

## Patentansprüche

1. Bildanzeigevorrichtung mit:
einer Eingabeeinheit (52), die konfiguriert ist, um durch ein in-vivo-Abbildungsgerät (10) in Zeitsequenz aufgenommene Bilddaten einzugeben;
einer Skalen-Anzeigesteuervorrichtung, die konfiguriert ist zum Steuern einer Anzeige einer Skala (507), die einen Gesamt-Abbildungszeitraum der in Zeitsequenz aufgenommenen und von der Eingabeeinheit (52) eingegebenen Bilddaten angibt, sowie zum Anzeigen eines beweglichen Gleitelements (S) auf der Skala (507); und
einer Bildanzeige-Steuervorrichtung, die konfiguriert ist zum Steuern einer Anzeige eines Bildes auf einer Anzeigeeinheit (51) zu einer Abbildungszeit, welche einer Position des Gleitelements entspricht, in Reaktion auf eine Bewegung des Gleitelements (S) auf der Skala (507),
**dadurch gekennzeichnet, dass** sie umfasst
eine Farbinformations-Erfassungseinheit, die konfiguriert ist zum Erfassen von Farbinformation einer Bildschirmanzeige der von der Eingabeeinheit eingegebenen Bilddaten, und die eine Durchschnittsfarben-Erfassungseinheit aufweist, die zum Erfassen einer Durchschnittsfarbe aus der Farbinformation konfiguriert ist;
eine Farbanzeige-Steuereinheit, die konfiguriert ist zum Anzeigen einer Farbe, die der von der Durchschnittsfarben-Erfassungseinheit erfassten Durchschnittsfarbe an einer der Zeit entsprechenden Position auf der Skala (507) entspricht;
eine Organ-Unterscheidungseinheit, die konfiguriert ist zum Unterscheiden eines Organs auf der Basis der von der Farbinformations-Erfassungseinheit erfassten Farbinformation; und
eine Organnamensanzeige-Steuereinheit, die konfiguriert ist zum Steuern einer Anzeige eines Namens (509) des von der Organ-Unterscheidungseinheit unterschiedenen Organs in Verbindung mit der Skala (507), so dass der Name (509) des Organs in Verbindung mit der Durchschnittsfarbe des Organs angezeigt wird.

2. Bildanzeigevorrichtung nach Anspruch 1, wobei die Organ-Unterscheidungseinheit konfiguriert ist, um das Organ basierend auf Information über eine Zunahme und Abnahme von die Farbinformation bildenden Farbelementen zu unterscheiden.

3. Bildanzeigevorrichtung nach Anspruch 1, wobei die Organ-Unterscheidungseinheit konfiguriert ist, um das Organ ferner basierend auf gemäß den Bilddaten gewonnener in-vivo-Information zu unterscheiden.

4. Bildanzeigeverfahren, umfassend:
einen Eingabeschritt zum Eingeben von durch ein in-vivo-Abbildungsgerät (10) in Zeitsequenz aufgenommenen Bilddaten;
einen Skalenanzeige-Steuerschritt zum Steuern einer Anzeige einer Skala (507) die einen Gesamt-Abbildungszeitraum der in Zeitsequenz aufgenommenen und in dem Eingabeschritt eingegebenen Bilddaten angibt, sowie einer Anzeige eines beweglichen Gleitelements (S) auf der Skala (507);
einen Bildanzeige-Steuerschritt zum Steuern einer Anzeige eines Bildes auf einer Anzeigeeinheit (51) zu einer Abbildungszeit, welche einer Position des Gleitelements (S) entspricht, in Reaktion auf eine Bewegung des Gleitelements (S) auf der Skala (507);
einen Farbinformations-Erfassungsschritt zum Erfassen von Farbinformation einer Bildschirmanzeige der in dem Eingabeschritt eingegebenen Bilddaten, wobei der Farbinformations-Erfassungsschritt einen Durchschnittsfarben-Erfassungsschritt zum Erfassen einer Durchschnittsfarbe aus der Farbinformation umfasst;
einen Farbanzeige-Steuerschritt zum Steuern einer Anzeige einer Farbe, die dem in dem Durchschnittsfarben-Erfassungsschritt erfassten Durchschnitt an einer der Zeit entsprechenden Position auf der Skala (507) entspricht;
einen Organ-Unterscheidungsschritt zum Unterscheiden eines Organs auf der Basis der in dem Farbinformations-Erfassungsschritt erfassten Farbinformation; und
einen Organnamensanzeige-Steuerschritt zum Steuern einer Anzeige eines Namens (509) des in dem Organ-Unterscheidungsschritt unterschiedenen Organs in Verbindung mit der Skala (507), so dass der Name (509) des Organs in Verbindung mit der Durchschnittsfarbe des Organs angezeigt wird.

5. Bildanzeigeverfahren nach Anspruch 4, wobei der Organ-Unterscheidungsschritt das Unterscheiden des Organs basierend auf Information über eine Zunahme und Abnahme von die Farbinformation bildenden Farbelementen umfasst.

6. Bildanzeigeverfahren nach Anspruch 4, wobei der Organ-Unterscheidungsschritt das Unterscheiden des Organs, ferner basierend auf gemäß den Bilddaten gewonnener in-vivo-Information, umfasst.

7. Bildanzeigeprogramm, das einen Computer ausführen lässt:
einen Eingabeschritt zum Eingeben von durch ein in-vivo-Abbildungsgerät (10) in Zeitsequenz aufgenommenen Bilddaten;
einen Skalenanzeige-Steuerschritt zum Steuern einer Anzeige einer Skala (507) die einen Gesamt-Abbildungszeitraum der in Zeitsequenz aufgenommenen und in dem Eingabeschritt eingegebenen Bilddaten angibt, sowie einer Anzeige eines beweglichen Gleitelements (S) auf der Skala (507);
einen Bildanzeige-Steuerschritt zum Steuern einer Anzeige eines Bildes auf einer Anzeigeeinheit (51) zu einer Abbildungszeit, welche einer Position des Gleitelements (S) entspricht, in Reaktion auf eine Bewegung des Gleitelements (S) auf der Skala (507);
einen Farbinformations-Erfassungsschritt zum Erfassen von Farbinformation einer Bildschirmanzeige der in dem Eingabeschritt eingegebenen Bilddaten, wobei der Farbinformations-Erfassungsschritt einen Durchschnittsfarben-Erfassungsschritt zum Erfassen einer Durchschnittsfarbe aus der Farbinformation umfasst;
einen Farbanzeige-Steuerschritt zum Steuern einer Anzeige einer Farbe, die der in dem Durchschnittsfarben-Erfassungsschritt erfassten Durchschnittsfarbe an einer der Zeit entsprechenden Position auf der Skala (507) entspricht;
einen Organ-Unterscheidungsschritt zum Unterscheiden eines Organs auf der Basis der in dem Farbinformations-Erfassungsschritt erfassten Farbinformation; und
einen Organnamensanzeige-Steuerschritt zum Steuern einer Anzeige eines Namens (509) des in dem Organ-Unterscheidungsschritt unterschiedenen Organs in Verbindung mit der Skala (507), so dass der Name (509) des Organs in Verbindung mit der Durchschnittsfarbe des Organs angezeigt wird.

8. Bildanzeigeprogramm nach Anspruch 7, wobei der Organ-Unterscheidungsschritt das Unterscheiden des Organs basierend auf Information über eine Zunahme und Abnahme von die Farbinformation bildenden Farbelementen umfasst.

9. Bildanzeigeprogramm nach Anspruch 7, wobei der Organ-Unterscheidungsschritt das Unterscheiden des Organs, ferner basierend auf gemäß den Bilddaten gewonnener in-vivo-Information, umfasst.

## Revendications

1. Appareil d'affichage d'images comprenant :
une unité d'entrée (52) configurée pour délivrer en entrée des données d'images prises en séquence chronologique par un dispositif de traitement d'image in vivo (10) ;
une unité de commande d'affichage d'échelle configurée pour commander l'affichage d'une échelle (507) indiquant une période de traitement d'image globale des données d'images prises en séquence chronologique et délivrées en entrée par l'unité d'entrée (52) et pour afficher un curseur de défilement mobile (S) sur l'échelle (507) ; et
une unité de commande d'affichage d'images configurée pour commander l'affichage sur une unité d'affichage (51) d'une image à un moment de traitement d'image correspondant à une position du curseur de défilement en réponse à un déplacement du curseur de défilement (S) sur l'échelle (507),
**caractérisé en ce qu'**il comprend
une unité de détection d'informations de couleur configurée pour détecter des informations de couleur d'un écran des données d'images délivrées en entrée par l'unité d'entrée, et comprenant une unité de détection de couleur moyenne configurée pour détecter une couleur moyenne à partir des informations de couleur ;
une unité de commande d'affichage de couleur configurée pour commander l'affichage d'une couleur correspondant à la couleur moyenne détectée par l'unité de détection de couleur moyenne au niveau d'une position sur l'échelle (507) correspondant au temps ;
une unité de discrimination d'organe configurée pour discriminer un organe sur la base des informations de couleur détectées par l'unité de détection d'informations de couleur ; et
une unité de commande d'affichage de nom d'organe configurée pour commander l'affichage d'un nom (509) de l'organe discriminé par l'unité de discrimination d'organe en association avec l'échelle (507) de sorte que le nom (509) de l'organe est affiché en association avec la couleur moyenne de l'organe.

2. Appareil d'affichage d'images selon la revendication 1, dans lequel l'unité de discrimination d'organe est configurée pour discriminer l'organe sur la base des informations sur l'augmentation et la diminution des éléments de couleur constituant les informations de couleur.

3. Appareil d'affichage d'images selon la revendication 1, dans lequel l'unité de discrimination d'organe est configurée pour discriminer l'organe en se basant en outre sur les informations in vivo obtenues conformément aux données d'images.

4. Procédé d'affichage d'images comprenant :
une étape d'entrée destinée à délivrer en entrée des données d'images prises en séquence chronologique par un dispositif de traitement d'image in vivo (10) ;
une étape de commande d'affichage d'échelle destinée à commander l'affichage d'une échelle (507) indiquant une période de traitement d'image globale des données d'images prises en séquence chronologique et délivrées en entrée à l'étape d'entrée et à afficher un curseur de défilement mobile (S) sur l'échelle (507) ;
une étape de commande d'affichage d'images destinée à commander l'affichage sur une unité d'affichage (51) d'une image à un moment de traitement d'image correspondant à une position du curseur de défilement (S) en réponse à un déplacement du curseur de défilement (S) sur l'échelle (507),
une étape de détection d'informations de couleur destinée à détecter des informations de couleur d'un écran des données d'images délivrées en entrée à l'étape d'entrée, dans lequel l'étape de détection d'informations de couleur comprend une étape de détection de couleur moyenne destinée à détecter une couleur moyenne à partir des informations de couleur ;
une étape de commande d'affichage de couleur destinée à commander l'affichage d'une couleur correspondant à la couleur moyenne détectée à l'étape de détection de couleur moyenne au niveau d'une position sur l'échelle (507) correspondant au temps ;
une étape de discrimination d'organe destinée à discriminer un organe sur la base des informations de couleur détectées à l'étape de détection d'informations de couleur ; et
une étape de commande d'affichage de nom d'organe destinée à commander l'affichage d'un nom (509) de l'organe discriminé à l'étape de discrimination d'organe en association avec l'échelle (507) de sorte que le nom (509) de l'organe est affiché en association avec la couleur moyenne de l'organe.

5. Procédé d'affichage d'images selon la revendication 4, dans lequel l'étape de discrimination d'organe comprend la discrimination de l'organe sur la base des informations sur l'augmentation et la diminution des éléments de couleur constituant les informations de couleur.

6. Procédé d'affichage d'images selon la revendication 4, dans lequel l'étape de discrimination d'organe comprend la discrimination de l'organe en se basant en outre sur des informations in vivo obtenues conformément aux données d'images.

7. Programme d'affichage d'images amenant un ordinateur à exécuter :
une étape d'entrée destinée à délivrer en entrée des données d'images prises en séquence chronologique par un dispositif de traitement d'image in vivo (10) ;
une étape de commande d'affichage d'échelle destinée à commander l'affichage d'une échelle (507) indiquant une période de traitement d'image globale des données d'images prises en séquence chronologique et délivrées en entrée à l'étape d'entrée et à afficher un curseur de défilement mobile (S) sur l'échelle (507) ;
une étape de commande d'affichage d'images destinée à commander l'affichage sur une unité d'affichage (51) d'une image à un moment de traitement d'image correspondant à une position du curseur de défilement (S) en réponse à un déplacement du curseur de défilement (S) sur l'échelle (507),
une étape de détection d'informations de couleur destinée à détecter des informations de couleur d'un écran des données d'images délivrées en entrée à l'étape d'entrée, dans lequel l'étape de détection d'informations de couleur comprend une étape de détection de couleur moyenne destinée à détecter une couleur moyenne à partir des informations de couleur ;
une étape de commande d'affichage de couleur destinée à commander l'affichage d'une couleur correspondant à la couleur moyenne détectée à l'étape de détection de couleur moyenne au niveau d'une position sur l'échelle (507) correspondant au temps ;
une étape de discrimination d'organe destinée à discriminer un organe sur la base des informations de couleur détectées à l'étape de détection d'informations de couleur ; et
une étape de commande d'affichage de nom d'organe destinée à commander l'affichage d'un nom (509) de l'organe discriminé à l'étape de discrimination d'organe en association avec l'échelle (507) de sorte que le nom (509) de l'organe est affiché en association avec la couleur moyenne de l'organe.

8. Programme d'affichage d'images selon la revendication 7, dans lequel l'étape de discrimination d'organe comprend la discrimination de l'organe sur la base des informations sur l'augmentation et la diminution des éléments de couleur constituant les informations de couleur.

9. Programme d'affichage d'images selon la revendication 7, dans lequel l'étape de discrimination d'organe comprend la discrimination de l'organe en se basant en outre sur des informations in vivo obtenues conformément aux données d'images.
